(19) Europäisches Patentamt / European Patent Office / Office européen des brevets

(11) **EP 3 964 834 B1**

(12) **EUROPÄISCHE PATENTSCHRIFT**

(45) Veröffentlichungstag und Bekanntmachung des Hinweises auf die Patenterteilung:
**14.06.2023   Patentblatt 2023/24**

(21) Anmeldenummer: **21204394.7**

(22) Anmeldetag: **03.12.2019**

(51) Internationale Patentklassifikation (IPC):
***G01N 33/28*** *(2006.01)*

(52) Gemeinsame Patentklassifikation (CPC):
**G01N 33/2894**

(54) **VERFAHREN UND VORRICHTUNG ZUR ÜBERWACHUNG EINER KÜHL-SCHMIERSTOFFEMULSION**

METHOD AND DEVICE FOR MONITORING A COOLING AND LUBRICATING EMULSION

PROCÉDÉ ET DISPOSITIF DE SUPERVISION D'UNE ÉMULSION DE LUBRIFIANT ET REFROIDISSEMENT

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **18.12.2018   EP 18213572**

(43) Veröffentlichungstag der Anmeldung:
**09.03.2022   Patentblatt 2022/10**

(62) Dokumentnummer(n) der früheren Anmeldung(en) nach Art. 76 EPÜ:
**19813812.5 / 3 899 529**

(73) Patentinhaber: **Blaser Swisslube AG**
**3415 Hasle-Rüegsau (CH)**

(72) Erfinder:
• **Katzenmeier, Heinz**
  **3432 Lützelflüh (CH)**
• **Schneeberger, Manfred**
  **3110 Münsingen (CH)**

(74) Vertreter: **Jacobi, Markus Alexander**
**Patentanwälte**
**Isenbruck Bösl Hörschler PartG mbB**
**Eastsite One**
**Seckenheimer Landstrasse 4**
**68163 Mannheim (DE)**

(56) Entgegenhaltungen:
EP-A2- 1 365 236       GB-A- 2 547 056
JP-A- H08 233 804      US-A- 4 315 421
US-A1- 2004 159 145    US-A1- 2007 202 603

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die Erfindung betrifft ein Verfahren zur Bestimmung mindestens eines Parameters und/oder zur Konditionierung einer Kühl-Schmierstoffemulsion für Werkzeugmaschinen, wobei die Kühl-Schmierstoffemulsion in einem Tank bereitgestellt wird, wobei Kühl-Schmierstoffemulsion aus dem Tank entnommen wird und unter Verwendung mindestens eines Sensors mindestens ein Parameter der Kühl-Schmierstoffemulsion erfasst wird. Ein weiterer Aspekt der Erfindung betrifft eine Vorrichtung, welche zur Durchführung des Verfahrens eingerichtet ist.

Stand der Technik

Bei einer spanenden Bearbeitung von Werkstücken wird diesen durch mechanisches Abtrennen von überschüssigem Material in Form von Spänen mit einem Werkzeug eine bestimmte Form gegeben. Die spanenden Fertigungsverfahren umfassen beispielsweise Drehen, Fräsen und Schleifen. Aufgrund von Reibung wird dabei die eingebrachte mechanische Arbeit fast vollständig in Wärme umgewandelt.

Kühl-Schmierstoffemulsionen, kurz KSS-Emulsionen, werden in der Metallverarbeitenden Industrie breit zur zerspanenden Metallbearbeitung eingesetzt. KSS-Emulsionen werden oftmals aus KSS-Konzentraten durch Einrühren in Wasser hergestellt. KSS-Konzentrate bestehen in der Regel aus einer Ölkomponente, Puffer-Komponenten, Emulgatoren und verschiedenen Additiven. KSS-Konzentrate sind homogene flüssige Produkte mit öliger Konsistenz.

Die Kühl-Schmierstoffemulsionen (KSS) werden eingesetzt, um das bearbeitete Werkstück und/oder das Werkzeug zu kühlen und zu schmieren. Dazu werden die Kühl-Schmierstoffemulsionen üblicherweise aus einem Tank gefördert und auf das Werkzeug bzw. das Werkstück aufgetragen. Die Kühl-Schmierstoffemulsion wird anschließend wieder aufgefangen und zurückgeführt.

Während des Einsatzes der Kühl-Schmierstoffemulsion wird diese aufgefangen und zusammen mit aus dem Zerspan-Prozess entnommenen Spänen ausgetragen. Die Kühl-Schmierstoffemulsion haftet dabei den ausgetragenen Spänen an. Vor einer erneuten Verwendung der Kühl-Schmierstoffemulsion muss diese somit von den Spänen und anderen Verunreinigungen befreit werden. Zudem erfolgt durch das feine Versprühen der Kühl-Schmierstoffemulsion bei den in den Maschinen eingesetzten, hohen Drücken eine Verdunstung von Wasser. Dies bewirkt eine Anreicherung der Aktivkomponenten wie Ölen, Additiven und dergleichen in der Kühl-Schmierstoffemulsion.

Zum Ausgleich der ausgetragenen Kühl-Schmierstoffemulsion und der Wasserverdunstung muss regelmäßig Kühl-Schmierstoffemulsion niedriger Konzentration nachgefüllt werden, was als "Nachfahren" bezeichnet wird. Die Nachfahrmenge und Konzentration lässt sich aus der aktuellen Ist- und Soll-Konzentration und der Füllstands-Differenz im Tank berechnen.

Aus GB 2547056 A ist ein System zum Bestimmen des Zustands einer Flüssigkeit in einem Tank bekannt. Das System umfasst einen oder mehrere Sensoren, mit denen jeweils eine Eigenschaft der Flüssigkeit bestimmt werden kann. Abhängig von den gemessenen Eigenschaften wird bestimmt, welche Menge eines Konzentrats und/oder welche Menge an Wasser zugegeben werden muss, um einen gewünschten Zustand der Flüssigkeit einzustellen.

US 5,389,546 beschreibt ein kontinuierliches Titrationsverfahren zum Bestimmen eines Alkaligehalts einer zur Metallbearbeitung eingesetzten Flüssigkeit. Bei dem Verfahren ist vorgesehen, kontinuierlich eine Probe der zur Metallbearbeitung eingesetzten Flüssigkeit zu entnehmen, mit einem kontinuierlich steigenden Strom einer Maßlösung zu vermischen und den pH-Wert kontinuierlich zu bestimmen. Aus dem Verhältnis der beiden Volumenströme lässt sich der Alkaligehalt berechnen. Der Flüssigkeitsstrom während der Messung wird dabei kontinuierlich entsorgt. Zur Kontrolle eines eingesetzten Sensors kann alternativ eine Probe mit bekanntem pH-Wert zugeführt werden.

US2004/159145 offenbart Messungen an einer Kühl-Schmierstoffemulsion, unter Verwendung eines Tanks, eines Sensorblocks, eines Einmischblocks und einer Förderpumpe. Der Sensorblock beinhaltet Sensoren zur Erfassung von Parametern, und der Einmischblock beinhaltet eine Additivpumpe.

Offenbarung der Erfindung

Es wird ein Verfahren zur Bestimmung mindestens eines Parameters einer Kühl-Schmierstoffemulsion für Werkzeugmaschinen vorgeschlagen. Die Kühl-Schmierstoffemulsion wird in einem Tank bereitgestellt. Bei dem Verfahren wird die Kühl-Schmierstoffemulsion aus dem Tank entnommen, unter Verwendung mindestens eines Sensors wird mindestens ein Parameter der Kühl-Schmierstoffemulsion erfasst und die Kühl-Schmierstoffemulsion wird zumindest teilweise in den Tank zurückgeführt. Das Verfahren umfasst ferner das Durchführen einer Messung, bei der eine Probe aus dem Tank entnommen wird, die Probe in einem Kreislauf fortlaufend an dem mindestens einen Sensor vorbeigeführt wird, um mindestens einen Parameter der Probe zu erfassen, wobei kontinuierlich oder schrittweise mindestens ein Mess-Reagenz zugegeben wird und der mindestens eine Parameter kontinuierlich gemessen wird, bis der mindestens eine Parameter einen vorgegebenen Schwellenwert erreicht, und anschließend zumindest ein Teil der Probe zurück in den Tank gefördert wird.

Optional kann das Verfahren zusätzlich einen Schritt des Kalibrierens des mindestens einen Sensors, durch Spülen des mindestens einen Sensors und Durchführen einer Messung mit frischer Kühl-Schmierstoffemulsion, welche ein definiertes Verhältnis eines Konzentrats zu Wasser aufweist, umfassen, der fortan als "Schritt i)" bezeichnet wird.

Dabei ist bei dem Kalibrieren gemäß dem optionalen Schritt i) vorgesehen, dass die Konzentration der Kühl-Schmierstoffemulsion in dem Tank gemessen wird, eine erforderliche Nachfahrkonzentration berechnet wird und die frische Kühl-Schmierstoffemulsion, welche für die Kalibrierung genutzt wird, mit der berechneten Nachfahrkonzentration bereitgestellt wird. Die frische Kühl-Schmierstoffemulsion wird dabei erhalten durch Fördern von Wasser und/oder Konzentrat unter Verwendung einer volumetrischen Pumpe und/oder durch Messen einer geförderten Menge von Wasser und/oder Konzentrat und Regeln einer Förderpumpe in Abhängigkeit der gemessenen geförderten Menge. Des Weiteren ist vorgesehen, dass die zur Kalibrierung genutzte Kühl-Schmierstoffemulsion nicht verworfen wird, sondern für eine Konditionierung der in dem Tank aufgenommenen Kühl-Schmierstoffemulsion verwendet wird. Entsprechend ist vorgesehen, den Tank mit der für die Kalibrierung verwendeten Kühl-Schmierstoffemulsion nachzufahren.

Bevorzugt ist der Tank mit mindestens einer Werkzeugmaschine verbunden, so dass Kühl-Schmierstoffemulsion aus dem Tank zu mindestens einer Werkzeugmaschine gefördert und von der mindestens einen Werkzeugmaschine zurück in den Tank gefördert werden kann.

Das vorgeschlagene Verfahren ermöglicht die besonders zuverlässige und exakte Bestimmung mindestens eines Parameters der Kühl-Schmierstoffemulsion. Bei dem mindestens einen Parameter kann es sich beispielsweise um einen Brechungsindex, einen pH-Wert, eine elektrische Leitfähigkeit, eine Lichtdurchlässigkeit oder einen Reflexionsgrad des Kühlschmierstoffs handeln. Des Weiteren können im Rahmen des Verfahrens Kombinationen mindestens zweier dieser Parameter gemessen werden. Aus dem bei einer Messung, bei der erfindungsgemäß eine oder mehrere Mess-Reagenzien verwendet werden, bestimmten mindestens einen Parameter können weitere Eigenschaften bestimmt werden. Abgeleitete Eigenschaften der Kühl-Schmierstoffemulsion können insbesondere ausgewählt sein aus einer Konzentration der Kühl-Schmierstoffemulsion, einem Total-Amin / Total-Säure Wert (TA/TS-Wert), einer Pufferkapazität, einem Metallgehalt, einem Ölgehalt und einem Nitrit-Gehalt. Insbesondere zur Berechnung der Nachfahrkonzentration ist eine zuverlässige Messung der Konzentration der Kühl-Schmierstoffemulsion im Tank notwendig. Üblicherweise wird zur Bestimmung der Konzentration der Kühl-Schmierstoffemulsion unter Verwendung eines Refraktometers der Brechungsindex der Kühl-Schmierstoffemulsion gemessen. Ein Refraktometer bestimmt den Brechungsindex an der Grenzfläche einer transparenten Oberfläche zur Kühl-Schmierstoffemulsion. Ein großes Problem besteht dabei in der Bildung von öligen Ablagerungen (Ölfilmen) aus den ölhaltigen Kühl-Schmierstoffemulsionen auf dem Messfenster. Diese Ablagerungen verursachen eine schleichende Erhöhung des Messwertes und suggerieren eine zu hohe Konzentration der Kühl-Schmierstoffemulsion im Tank. Dies wiederum führt zur fehlerhaften Berechnung der Konzentration der zum Nachfahren bereitgestellten frischen Kühl-Schmierstoffemulsion, welche dabei insbesondere zu gering gewählt wird.

Dies wiederum führt dazu, dass die Konzentration der Kühl-Schmierstoffemulsion im Tank niedriger ist als beabsichtigt, was Probleme verursacht, wie eine Verringerung der Zerspan-Leistung, erhöhter Werkzeug-Verschleiß, Korrosion und Anfälligkeit gegenüber mikrobiologischem Befall.

Da die fehlerhafte Messung des Refraktometers schleichend erfolgt ist sie schwierig festzustellen. Meist wird hierzu eine Kontrollmessung mit einem zweiten Refraktometer durchgeführt. Alternativ kann dem Messsystem eine Kalibrierlösung zugeführt werden, welche die Messgenauigkeit des Refraktometers überprüft. Die verwendeten Kalibrierlösungen müssen in der Regel wieder aus dem System ausgeschleust und entsorgt werden. Ein einfaches abfallfreies Verfahren, mit dem der Verschmutzungszustand des Refraktomers jederzeit festgestellt werden kann wäre wünschenswert.

Daher ist gemäß optionalem Schritt i) des Verfahrens vorgesehen, den mindestens einen Sensor mit frischer Kühl-Schmierstoffemulsion zu spülen und im Anschluss eine Messung des mindestens einen Parameters dieser frischen Kühl-Schmierstoffemulsion unter Verwendung dieses Sensors auszuführen. Da die frische Kühl-Schmierstoffemulsion ein definiertes Verhältnis eines Konzentrats zu Wasser aufweist, sind deren Eigenschaften bekannt, so dass der erhaltene Messwert für den mindestens einen Parameter der Kühl-Schmierstoffemulsion mit einem vorab bestimmten Sollwert verglichen werden kann.

Durch Vergleich des Messwerts für den mindestens einen Parameter der frischen Kühl-Schmierstoffemulsion mit dem vorab bestimmten Sollwert kann eine Kalibrierung des mindestens einen Sensors erfolgen. Dazu kann beispielsweise ein Korrekturfaktor bestimmt werden, mit dem Messwerte des mindestens einen Sensors korrigiert werden. Sollte keine Kalibrierung möglich sein oder eine Abweichung zwischen dem Messwert und dem Sollwert größer als ein vorgegebener Grenzwert sein, kann auch eine Warnmeldung ausgegeben werden, um anzuzeigen, dass der Sensor gewartet oder ersetzt werden muss.

Beispielsweise ist der Brechungsindex einer frischen Kühl-Schmierstoffemulsion mit einem genau definierten Verhältnis von Wasser zu Konzentrat bekannt, so dass diese Emulsion zur Kalibrierung eines Refraktometers herangezogen werden kann. Im Gegensatz zu einer von der Kühl-Schmierstoffemulsion verschiedenen Kalibrierflüssigkeit kann die Kühl-Schmierstoffemulsion anschließend in den Tank geleitet werden, so dass keine Abfälle entstehen. Es muss auch keine separate Kalibrierflüssigkeit und/oder eine separate Reinigungsflüssigkeit vorrätig gehalten werden.

Gemäß dem vorgeschlagenen Verfahren wird die für die Kalibrierung verwendete frische Kühl-Schmierstoffemulsion nicht verworfen, sondern in den Tank geleitet, so dass der Tank unter Verwendung der frischen Kühl-Schmierstoffemulsion nachgefahren wird.

Die für die Kalibrierung bereitgestellte frische Kühl-Schmierstoffemulsion weist ein bekanntes Verhältnis zwischen Konzentrat und Wasser auf. Dieses Verhältnis ist jedoch von der momentan gemessenen Konzentration der Kühl-Schmierstoffemulsion abhängig. Bevorzugt wird die vorgeschlagene Kalibrierung immer dann durchgeführt, wenn der Tank nachgefahren werden muss. Entsprechend wird für die Kalibrierung immer eine frische Kühl-Schmierstoffemulsion verwendet, deren Konzentration bekannt ist, jedoch in der Regel bei jedem Kalibrierungsvorgang einen anderen Wert aufweist. Somit kann der Sensor mit verschiedenen Kühl-Schmierstoffemulsionen als Testflüssigkeit kalibriert werden, wobei die Eigenschaften dieser verschiedenen Kühl-Schmierstoffemulsionen jeweils bekannt sind. Dies erlaubt es insbesondere, zwischen einer verringerten Empfindlichkeit des Sensors und einer Verschiebung eines Nullpunkts des Sensors zu unterscheiden.

Typischerweise enthält die für die Kalibrierung und für das Nachfahren des Tanks bereitgestellte frische Kühl-Schmierstoffemulsion einen Ölanteil im Bereich von 0% bis 10%, wobei aufgrund der Abhängigkeit von der im Tank gemessenen Konzentration bei jedem Nachfahren in der Regel eine andere Konzentration in der frischen Kühl-Schmierstoffemulsion eingestellt wird. Ein Anteil des Öls im Bereich von 0% bis 10% ist für das Kalibrieren optimal, da bei diesen Konzentrationen kein bleibender Ölfilm auf dem Sensor verbleibt.

Außer dem Brechungsindex werden bevorzugt noch weitere Parameter zur Beschreibung des ordnungsgemäßen Zustands der Kühl-Schmierstoffemulsion ermittelt. Besonders wichtig sind hierzu der pH-Wert, die Pufferkapazität und die Leitfähigkeit. Zur Messung des pH-Wertes und der Leitfähigkeit werden bevorzugt Messelektroden eingesetzt. Auch diese Messelektroden neigen wie das zuvor beschriebene Refraktometer zum Verölen. Dabei ändern sich schleichend die Messwerte was zu unerwünschten und schwer feststellbaren Fehlmessungen führt. Auch hier wird daher bevorzugt ein Spülen und/oder Kalibrieren unter Verwenden einer frischen Kühl-Schmierstoffemulsion mit definierter Konzentration durchgeführt.

Die frische Kühl-Schmierstoffemulsion mit der definierten Konzentration bzw. dem definierten Verhältnis von Konzentrat zu Wasser wird bevorzugt erhalten durch Fördern von Wasser und/oder Konzentrat unter Verwendung einer volumetrischen Pumpe. Alternativ oder zusätzlich kann die frische Kühl-Schmierstoffemulsion erhalten werden durch Fördern von Konzentrat und/oder Wasser mit einer regelbaren Förderpumpe, wobei die geförderten Menge von Wasser und/oder Konzentrat gemessen wird und die Förderpumpe in Abhängigkeit der gemessenen geförderten Menge geregelt wird.

Das Herstellen von frischer Kühl-Schmierstoffemulsion mit einer definierten Konzentration wird bevorzugt nicht nur für ein Spülen und/oder Kalibrieren des mindestens einen Sensors verwendet, sondern immer dann, wenn zum Nachfahren frische Kühl-Schmierstoffemulsion benötigt wird. Umgekehrt erfolgt ein Spülen und Kalibrieren des mindestens einen Sensors bevorzugt bei jedem Nachfahren mit frischer Kühl-Schmierstoffemulsion mit einer definierten Konzentration.

Die definierte Konzentration wird bevorzugt in Abhängigkeit der gemessenen Konzentration der im Tank bevorrateten Kühl-Schmierstoffemulsion derart festgelegt, dass beim Nachfahren mit der frischen Kühl-Schmierstoffemulsion mit der zum Auffüllen des Tanks benötigten Menge sich im Tank eine Konzentration der Kühl-Schmierstoffemulsion einstellt, die einer Vorgabe entspricht.

Bestimmte Inhaltsstoffe wie beispielsweise Öle, oberflächenaktive Additive, Amine, Fettsäuren, Korrosionsinhibitoren, Leistungsadditive, Stabilisatoren und/oder Biozide werden beim Abtrennen von Spänen von der Kühl-Schmierstoffemulsion überproportional aus der Kühl-Schmierstoffemulsion ausgetragen. Dies führt dazu, dass die Kühl-Schmierstoffemulsion an diesen Inhaltsstoffen verarmt. Diese Verarmung an Inhaltsstoffen führt zu Qualitätseinbußen wie eine Verringerung der Zerspanleistung oder eine Erhöhung der Anfälligkeit gegenüber Korrosion oder mikrobiellen Befall. Die selektive Verarmung der Kühl-Schmierstoffemulsion kann durch Zusatz von frischer Kühl-Schmierstoffemulsion nicht ausgeglichen werden. Bevorzugt kann aus dem mindestens einen Parameter der Kühl-Schmierstoffemulsion auf die Verarmung eines Inhaltsstoffes geschlossen werden. Wird eine solche Verarmung erkannt, ist bevorzugt vorgesehen, selektiv die ausgetragenen Komponenten durch die Zugabe von Additiven zu ersetzen.

Erfindungsgemäß wird eine Messung durchgeführt, bei der eine Probe der Kühl-Schmierstoffemulsion aus dem Tank entnommen wird, die Probe in einem Kreislauf fortlaufend an dem mindestens einen Sensor vorbeigeführt wird, um mindestens einen Parameter der Probe zu erfassen, wobei kontinuierlich oder schrittweise mindestens ein Mess-Reagenz zugegeben wird und der mindestens eine Parameter kontinuierlich gemessen wird, bis der mindestens eine Parameter einen vorgegebenen Schwellenwert erreicht, und anschließend zumindest ein Teil der Probe zurück in den Tank gefördert wird.

Somit insbesondere eine Titration durchgeführt werden, bei der jeweils in kleinen Schritten eine Maßlösung als Mess-Reagenz zudosiert wird und anschließend abgewartet wird, bis sich das Mess-Reagenz homogen in der Emulsion verteilt hat. Anschließend wird jeweils mindestens ein Parameter der Kühl-Schmierstoffemulsion gemessen. Das Zudosieren, Abwarten und Messen wird solange wiederholt, bis für den mindestens einen Parameter ein vordefinierter Wert erreicht wird. Anschließend kann das Gesamtvolumen des zudosierten Mess-Reagenz bestimmt werden und aus diesem Gesamtvolumen auf eine Eigenschaft der Kühl-Schmierstoffemulsion geschlossen werden.

Im Kreislauf befindet sich mindestens eine Zugabestelle zur Zudosierung mindestens eines Additives und/oder eines Mess-Reagenz sowie mindestens ein Sensor der eingerichtet ist, eine Reaktion der im Kreislauf geführten Kühl-Schmierstoffemulsion zur Zudosierung des Additives bzw. der Mess-Reagenz zu detektieren. Nach der Messung wird das mit dem Additiv und/oder Mess-Reagenz versetzte Volumen zumindest teilweise der Kühl-Schmierstoffemulsion dem Tank zugeschlagen, so dass bei dem Verfahren keine Abfälle entstehen oder zumindest reduziert werden.

In einer Variante des Verfahrens kann ein Teil der Probe als Abfall verworfen und nicht in den Tank zurückgeführt werden. Bevorzugt wird das entnommene Volumen der Kühl-Schmierstoffemulsion vollständig in den Tank zurückgeführt. Hierbei werden bevorzugt Mess-Reagenzien eingesetzt, welche die Eigenschaften der Kühl-Schmierstoffemulsion nicht nachteilig beeinflussen und daher nach Abschluss des Messvorgangs zusammen mit der untersuchten Kühl-Schmierstoffemulsion in den Tank zurückgegeben werden können.

Bevorzugt wird unter Verwendung eines Agitators eine Vermischung zwischen der im Kreislauf geführten Kühl-Schmierstoffemulsion und einem zugeführten Additiv und/oder einem zugeführten Mess-Reagenz durchgeführt.

Bei dem Agitator kann es sich beispielsweise um einen Mischer handeln, wie einen statischer Mischer, um eine Vermischung der Bestandteile zu fördern. Zusätzlich oder alternativ können weitere Agitatoren wie ein Heizelement oder eine Bestrahlungseinheit eingesetzt werden, um die Kühl-Schmierstoffemulsion zu erwärmen oder zu bestrahlen, um eine chemische Reaktion der Bestandteile zu fördern.

Bevorzugt erfolgt vor dem kontinuierlichen Zugeben der Messreagenz mindestens eine diskontinuierliche Zugabe einer weiteren Messreagenz. Bevorzugt erfolgt dies einmalig zu Beginn einer Messung.

Erfindungsgemäß ist vorgesehen, eine Probe bzw. einen kleinen Teilstrom der Kühl-Schmierstoffemulsion in einem Kreislauf zu führen. Um das Volumen der Probe bei Zugabe der Messreagenz und/oder der weiteren Messreagenz konstant zu halten, wird bevorzugt ein Teil der Probe aus dem Kreislauf entnommen und in den Tank zurückgeführt. Dazu ist der Kreislauf ist bevorzugt offen zum Tank, so dass sich die Volumenänderung durch Zusatz der Additive zum Tank ausdehnen kann und nicht zu einem Druckanstieg im Messkreislauf führt.

Bevorzugt wird bei der Messung eine Messung einer Pufferkapazität der Kühl-Schmierstoffemulsion durchgeführt, wobei das Mess-Reagenz eine Maßlösung ausgewählt ist aus einer Säure oder einer Lauge und der mindestens eine Parameter der pH-Wert ist. Die Titration erfolgt erfindungsgemäß, indem ein kleines Volumen der Kühl-Schmierstoffemulsion bis zum Abschluss der Messung im Kreis geführt wird. Das Emulsionsvolumen durchströmt dabei sowohl einen Einmischblock, über den Mess-Reagenzien und Additive zudosiert werden können, als auch einen Sensorblock, welcher mindestens einen Sensor zur Messung mindestens eines Parameters der Kühl-Schmierstoffemulsion umfasst.

Bevorzugt wird bei der Messung eine Messung des Total-Amin / Total-Säure Wertes (TA/TS-Wert) durchgeführt, wobei das weitere Messreagenz eine Säure ist und eine Base als Messreagenz kontinuierlich zugegeben wird und wobei der mindestens eine Parameter der pH-Wert des Kühlschmierstoffs ist.

In Erweiterung zur Pufferkapazität gibt auch der Parameter "Total Amin / Total Säure" (der sogenannte TA/TS-Wert) weitere wichtige Hinweise zum Zustand der Kühl-Schmierstoffemulsion. Der TA/TS-Wert ist ein Maß für die Gesamtheit aller sauren und basischen Komponenten in der Kühlschmierstoffemulsion. Verringert sich die Menge an Base oder an Säure, zeigt sich dies in einer Verringerung des TA, respektive des TS Wertes. Eine Verringerung der Menge an Säure oder Base in der Kühl-Schmierstoffemulsion kann durch Austrag über die Späne oder durch Verunreinigung mit Fremdöl erfolgen und hat nachteilige Auswirkungen auf die Leistungsfähigkeit der Kühlschmierstoffemulsion. Beispielsweise wird der Korrosionsschutz verringert oder die Anfälligkeit gegenüber mikrobiologischen Befall steigt. Daher ist die zeitnahe Messung des TA/TS-Wertes zur frühzeitigen Erkennung sich anbahnender Probleme äußerst wichtig.

Zur Messung des Total-Amin / Total-Säure Wertes (TA/TS-Wert) wird dem zu analysierenden Teilstrom der Kühl-Schmierstoffemulsion vor der Titration eine bestimmte Menge an Säure, vorzugsweise eine organische Säure wie Ameisensäure, Essigsäure, Propionsäure oder anorganische Säuren wie Phosphorsäure, Phosphonsäure oder deren saure Ester zugesetzt. Die Titration erfolgt dann z.B. mit einer Base wie Kalilauge, Natronlauge oder einem Amin oder Amin-Lösung.

Bevorzugt wird bei der Messung ein Metallgehalt der Kühl-Schmierstoffemulsion bestimmt, wobei das Mess-Reagenz ausgewählt ist aus einem Komplexbildner und der mindestens eine Parameter eine Lichtdurchlässigkeit oder ein Reflexionsgrad des Kühlschmierstoffs für Licht ist.

Dabei sind Komplexbildner Substanzen, welche mit den nachzuweisenden Metallen reagieren und dabei insbesondere Komplexe bilden, deren Absorption elektromagnetischer Strahlung sich bei der Reaktion ändert, so dass deren Gehalt beispielsweise über einen Photometer detektiert werden kann.

Bevorzugt wird bei der Messung ein Ölgehalt der Kühl-Schmierstoffemulsion bestimmt, wobei das Mess-Reagenz ausgewählt ist aus einer konzentrierten Salzlösung oder eine Säure und über den mindestens einen Sensor der Anteil der sich abscheidenden Ölphase in der Kühl-Schmierstoffemulsion erkannt wird. Bevorzugt erfolgt bei der Messung zudem ein Erhitzen des Emulsionsvolumens, beispielsweise mit einem Heizelement.

Bevorzugt wird bei der Messung ein Nitritgehalt der Kühl-Schmierstoffemulsion bestimmt. Das verwendete Mess-Reagenz enthält dazu beispielsweise 1-Naphthylamin, Sulfanilsäure oder N-(1-Naphthyl)-ethylendi-amin. Der mindestens eine Parameter ist eine Durchlässigkeit der Kühl-Schmierstoffemulsion für Licht.

Bevorzugt wird bei dem optionalen Schritt i) frische Kühl-Schmierstoffemulsion durch Fördern eines Konzentrats aus einem Konzentratvorratsbehälter und Mischen des Konzentrats mit Wasser bereitgestellt. Für einen zuverlässigen Betrieb ist es dabei wünschenswert, ein Leerlaufen eines Konzentratbehälters frühzeitig erkennen zu können. Bevorzugt ist daher vorgesehen, dass bei dem optionalen Schritt i) eine Messung eines Füllpegels des Konzentrats im Konzentratbehälter bestimmt wird. Fällt der Füllpegel unter einen vorgegebenen Mindestpegel kann beispielsweise eine Meldung ausgegeben werden, die zu einem Wechsel des Konzentratbehälters auffordert. Des Weiteren kann beispielsweise vorgesehen sein, dass bei Erkennen eines leeren Konzentratbehälters eine Abschaltung erfolgt oder auf einen weiteren Konzentratbehälter umgeschaltet wird.

Bevorzugt wird zur Bereitstellung von frischer Kühl-Schmierstoffemulsion das Konzentrat über eine Konzentrat-Ansaugleitung mit einem Steigrohr aus dem Konzentratvorratsbehälter gefördert, wobei ein Füllpegel des Konzentrats im Konzentratvorratsbehälter bestimmt wird durch Einleiten von Druckluft in die Konzentrat-Ansaugleitung, wobei ein Luftdruck in der Konzentrat-Ansaugleitung mit einem Drucksensor gemessen wird, ein Grenzdruck für einen Druckanstieg bestimmt wird und unter Verwendung des Grenzdrucks der Füllpegel im Konzentratvorratsbehälter berechnet wird.

Bevorzugt erfolgt bei dem optionalen Schritt i) eine Konditionierung von im Tank aufgenommener Kühl-Schmierstoffemulsion, indem Kühl-Schmierstoffemulsion aus dem Tank entnommen wird, in Abhängigkeit des mindestens einen Parameters mindestens ein Additiv zur Kühl-Schmierstoffemulsion zugegeben wird und die konditionierte Kühl-Schmierstoffemulsion in den Tank zurückgeführt wird. Des Weiteren ist es möglich, vor dem Zurückführen in den Tank die Kühl-Schmierstoffemulsion unter Verwendung eines Mischers durchzumischen.

Das Additiv ist insbesondere ausgewählt aus einer Komponente der Kühl-Schmierstoffemulsion wie beispielsweise eine Entschäumerkomponente wie übliche zur Entschäumung eingesetzte Polysiloxane, Triisobutylphosphat, Wachsentschäumer oder mineralölbasierende Entschäumer, einer härtebildenden Komponente wie beispielsweise Calcium- und/oder Magnesiumsalze wie Calciumacetat, Magnesiumacetat, Calciumsulfonat, Magnesiumsiulfonat und deren Lösungen, eine korossionsinhibierenden Komponente wie Phosphor- oder phosphonsäureester und deren Salze, Triazinderivate und deren Salze, Carbonsäuren, Fettsäuren, di- oder multivalente Carbonsäuren und deren Neutralisationsprodukte, eine Buntmetallinhibierungskomponente wie Triazolderivate und deren Salze, eine Aluminium-inhibierende Komponente wie Silanderivate wie Tetraethylorthosilikat oder Meta-silikatlösungen, eine Biozidkomponente wie Butylbenzothiazolinon, Natriumomadin-Lösung eine Ölkomponente, eine Amin-Komponente, eine Puffer-Komponente oder ein Emulgator. Bei den genannten Additiven können jeweils auch deren Lösungen in Wasser, Öl oder anderen geeigneten Lösungsmitteln eingesetzt werden.

Bei dem Additiv kann es sich zudem um unverdünntes Konzentrat zur Herstellung einer Kühl-Schmierstoffemulsion handeln, wobei die Menge des Konzentrats in Abhängigkeit des mindestens einen Parameters bestimmt wird. Unverdünntes Konzentrat wird der Kühl-Schmierstoffemulsion insbesondere dann zudosiert, wenn festgestellt wird, dass die Konzentration der Kühl-Schmierstoffemulsion im Tank zu gering ist und der Füllpegel im Tank hoch ist, so dass eine Erhöhung des Volumens durch das Zugeben frischer Kühl-Schmierstoffemulsion mit höherer Konzentration zu einer unerwünschten Volumenzunahme führen würde.

Ein weiterer Aspekt der Erfindung betrifft die Bereitstellung einer Vorrichtung zur Bestimmung mindestens eines Parameters einer Kühl-Schmierstoffemulsion für Werkzeugmaschinen. Die Vorrichtung ist zur Ausführung eines der hierin beschriebenen Verfahren eingerichtet, so dass im Rahmen eines der Verfahren beschriebene Merkmale auch für die Vorrichtung gelten und umgekehrt im Rahmen der Vorrichtung beschriebene Merkmale auch für die Verfahren gelten.

Die Vorrichtung zur Bestimmung mindestens eines Parameters einer Kühl-Schmierstoffemulsion für Werkzeugmaschinen umfasst einen Sensorblock, umfassend mindestens einen Sensor, einen Einmischblock umfassend wenigstens eine Additivpumpe zum Einmischen eines Additivs oder eines Mess-Reagenz und eine Förderpumpe, um Kühl-Schmierstoffemulsion aus einem Tank zum Sensorblock und dem Einmischblock zu führen.

Ferner kann für die Ausführung des optionalen Schritts i) vorgesehen sein, dass die Vorrichtung zusätzlich eine Bereitstellungseinheit zum Breitstellen von frischer Kühl-Schmierstoffemulsion umfasst, welche eingerichtet ist, eine definierte Menge eines Konzentrats mit einer definierten Menge Wasser zu mischen, wobei die Vorrichtung eingerichtet ist, in einem Kalibriermodus der Vorrichtung frische Kühl-Schmierstoffemulsion durch den Sensorblock zu leiten, um den Sensorblock zu spülen und/oder eine Kalibrierung des mindestens einen Sensors vorzunehmen.

Erfindungsgemäß ist vorgesehen, dass die Vorrichtung eine Bypassleitung umfasst, über die in einem Messmodus der Vorrichtung Kühl-Schmierstoffemulsion, die den Sensorblock und den Einmischblock durchlaufen hat, zurück zur Förderpumpe geführt werden kann, wodurch ein von einem Tank abgetrenntes Probenvolumen der Kühl-Schmierstoffemulsion umgewälzt wird.

Die Vorrichtung kann ferner insbesondere eine Steuereinheit umfassen, welche eingerichtet ist, eines der beschriebenen Verfahren auszuführen. Die Vorrichtung ist dabei insbesondere ausgebildet, die Verfahren automatisiert ohne den Eingriff eines Nutzers auszuführen. Ferner kann die Vorrichtung Verbindungen zu Füllpegelsensoren aufweisen, um den Füllpegel von Kühl-Schmierstoffemulsion in einem Tank oder mehreren Tanks zu erfassen. Zusätzlich kann die Steuereinheit eingerichtet sein, abhängig vom Füllpegel und gegebenenfalls einer ermittelten Konzentration einer Kühl-Schmierstoffemulsion in einem Tank zu bestimmen, welche Menge einer frischen Kühl-Schmierstoffemulsion mit welcher Konzentration zum Nachfahren des Tanks erforderlich ist.

Geeignete Füllstandsensoren, welche mit der Vorrichtung verbunden werden können, umfassen beispielsweise Schwimmer oder auf Ultraschall basierende Sensoren. Des Weiteren können Sensoren genutzt werden, welche auf der TDR-Technologie (Time Domain Reflectometry) basieren.

Der Einmischblock der Vorrichtung umfasst mindestens einen Additivtank und eine Additivpumpe. Der Additivtank nimmt ein Additiv oder ein Mess-Reagenz auf. Die Additivpumpe ist bevorzugt als eine volumetrischen Pumpe, wie beispielsweise eine Peristaltikpumpe, ausgestaltet oder eine regelbare Pumpe in Verbindung mit einem Durchflussmesser. Somit kann die Menge des einzudosierenden Additivs bzw. Mess-Reagenz exakt vorgegeben werden.

Der Sensorblock umfasst mindestens einen Sensor. Der mindestens eine Sensor ist bevorzugt ausgewählt aus der Gruppe umfassend pH-Wert-Sensoren, Leitfähigkeitssensoren, Photometern, Lichtschranken und Refraktometern.

Ein Photometer umfasst einer Lichtquelle und einem Lichtsensor sowie gegebenenfalls einen oder mehrerer optischer Filter, um die zur Messung geeignete Wellenlänge zu Verfügung zu stellen. Alternativ zu einem optischen Filter kann eine monochromatische Lichtquelle wie LED, Laser und/oder ein wellenlängensensitiver Lichtsensor eingesetzt werden. Der Betrieb des Photometers kann in Reflexion oder Durchlicht erfolgen.

Ein Leitfähigkeitssensor umfasst beispielsweise zwei Elektroden, welche mit der zu untersuchenden Emulsion in Verbindung gebracht werden. Zwischen den Elektroden fließt ein elektrischer Strom, der abhängig ist von der elektrischen Leitfähigkeit der Emulsion.

Ein pH-Wert-Sensor umfasst üblicherweise eine Elektrode, insbesondere eine pH-Elektrode. An der pH-Elektrode bildet sich ein elektrisches Potential aus, welches abhängig ist von der Konzentration von H+ Ionen.

Eine Lichtschranke umfasst eine Lichtquelle und einen Detektor, wobei der Detektor eine Änderung der Transmission oder Reflexion des Lichtstrahls, beispielsweise durch Öltröpfchen oder größere Ölkompartimente, erkennt.

Ein Refraktometer bestimmt den Brechungsindex an der Grenzfläche einer transparenten Oberfläche zur Kühl-Schmierstoffemulsion.

Ein im Zusammenhang mit der Vorrichtung eingesetztes Refraktometer weist bevorzugt ein Messfenster und eine zum Messfenster führende Zulaufleitung auf, wobei der Querschnitt d1 der zu dem Messfenster führenden Zulaufleitung bevorzugt derart gewählt ist, dass die Strömungsgeschwindigkeit am Messfenster bei einem vorgegebenen Volumenstrom eine Strömungsgeschwindigkeit im Bereich von 1 bis 500 m/min liegt und die zum Messfenster führende Zulaufleitung zu einer Senkrechten zum Messfenster einen Winkel $\alpha$ im Bereich von 15 bis 75 ° einschließt. Besonders bevorzugt liegt die Strömungsgeschwindigkeit im Bereich von 5 bis 100 m/min und ganz besonders bevorzugt im Bereich von 10 bis 50 m/min. Durch die Wahl des Durchmessers der Zulaufleitung im Verhältnis zum erwarteten Durchfluss wird erreicht, dass das Messfenster von der in der Zulaufleitung geführten Emulsion mit hoher Geschwindigkeit angeströmt wird. In Kombination mit dem Winkel $\alpha$ der Zulaufleitung, der den Anströmwinkel definiert, wird eine Selbstreinigung des Refraktometers erreicht. Die Emulsion, welche das Messfenster anströmt, nimmt gegebenenfalls vorhandene Verunreinigungen auf dem Messfenster mit, ähnlich wie bei einem Hochdruckreiniger.

Das Refraktometer ist bevorzugt für einen erwarteten Volumenstrom bzw. Durchfluss im Bereich von 1 bis 10 l/min eingerichtet. Besonders bevorzugt ist der erwartete Volumenstrom im Bereich von 2 bis 8 l/min. Beispielsweise wird als Volumenstrom 4 l/min vorgegeben.

In einer Ausführungsform ist die Vorrichtung zur Verbindung mit genau einem Tank eingerichtet. Alternativ dazu ist die Vorrichtung zur Verbindung mit zwei oder mehr Tanks eingerichtet. In diesem Fall sind bevorzugt zwei bis zehn Verbindungen vorgesehen, so dass die Vorrichtung mit einer entsprechenden Anzahl von Tanks verbindbar ist.

Bevorzugt umfasst die Vorrichtung einen Verbindungsblock, welcher mit der Förderpumpe in Verbindung steht, und eine Vielzahl von Ansaugleitungen für eine Vielzahl von Tanks aufweist, so dass die Vorrichtung selektiv mit jeweils einem Tank aus der Vielzahl von Tanks verbunden werden kann. Zur Rückführung der Kühl-Schmierstoffemulsion ist bevorzugt eine entsprechende Anzahl an Rückführleitungen vorgesehen.

Insbesondere bei Ausführungsformen der Vorrichtung, welche mit mehr als einem Tank verbindbar sind, ist es bevorzugt Maßnahmen zu treffen, um eine Vermischung verschiedener Kühl-Schmierstoffemulsionen zu vermeiden. Daher ist bevorzugt eine Druckluftleitung vorgesehen ist, welche über ein Druckluftventil mit einer Ansaugleitung verbunden ist, so dass in der Ansaugleitung und/oder dem Sensorblock und/oder in dem Einmischblock vorhandene Kühl-Schmierstoffemulsion ausgeblasen werden kann. Durch die über die Druckluftleitung eingeleitete Druckluft wird gegebenenfalls vorhandene Kühl-Schmierstoffemulsion in den jeweiligen Tank zurückgedrückt. Dies ist besonders vorteilhaft, da dadurch auch sich im Tank in der Ansaugleitung befindliche Spänefilter, welche verhindern, dass Späne und Partikel in den Messkreislauf gelangen, ausgeblasen und dadurch gereinigt werden.

Neben dem kurzen Ausblasen zu Reinigungszwecken kann über die Druckluftleitung zudem eine länger andauernde Belüftung des Tanks erfolgen. Auf diese Weise kann insbesondere bei langen Standzeiten der Bildung schlechter Gerüche vorgebeugt werden, indem ein anaerober Zustand der Emulsion durch den Sauerstoffeintrag verhindert wird.

Bevorzugt umfasst die Bereitstellungseinheit, die für die Ausführung des optionalen Schritts i) vorhanden sein kann, mindestens eine volumetrische Pumpe, um das Wasser und/oder das Konzentrat zu fördern. Alternativ oder zusätzlich umfasst die Bereitstellungseinheit mindestens eine regelbare Pumpe und einen der Pumpe zugeordneten Durchflussmesser, mit dem ein durch die Pumpe gefördertes Volumen bestimmt wird, wobei die Bereitstellungseinheit eingerichtet ist, die Pumpe abhängig vom geförderten Volumen zu regeln. Dabei sind verschiedene Kombinationen denkbar. Beispielsweise kann Wasser unter Verwendung einer geregelten Pumpe gefördert werden und das Konzentrat kann über eine volumetrische Pumpe gefördert werden. Geeignete Volumetrische Pumpen umfassen beispielsweise Peristaltikpumpen, Zahnradpumpen und Kolbenpumpen. Des Weiteren ist es denkbar, dass insbesondere zum Fördern des Wassers ein regelbares Ventil in Verbindung mit einem Durchflussmesser eingesetzt wird, wobei das Ventil in Abhängigkeit des gemessenen Durchflusses geregelt wird.

Üblicherweise muss das Wasser nicht gepumpt werden, da es aus der Leitung kommt. Vom Wasser wird dementsprechend bevorzugt nur der Durchfluss gemessen und nicht zusätzlich geregelt. Das Konzentrat wird dann mittels einer volumetrischen Pumpe oder mit einer regelbaren Pumpe und einem Durchflussmesser dem Wasserfluss angepasst.

Die Bereitstellungseinheit kann ferner einen Mischer umfassen. Bevorzugt umfasst die Bereitstellungseinheit einen statischen Mischer zur Vermischung von Wasser und Konzentrat. Über den Mischer wird eine gute Durchmischung des geförderten Konzentrats mit dem Wasser erzielt, so dass eine homogene Emulsion bereitgestellt wird.

Bevorzugt umfasst die Vorrichtung des Weiteren mindestens einen Agitator, welcher in Strömungsrichtung der Kühl-Schmierstoffemulsion dem Einmischblock nachgeschaltet ist, wobei der Agitator ausgewählt ist aus einem Mischer, einem Heizelement, einem Kühlelement, einer Bestrahlungseinheit und Kombinationen aus mehreren dieser Agitatoren. Dabei ist es möglich, über entsprechende Leitungen und Ventile für den Mischer der Bereitstellungseinheit als auch für den Agitator dasselbe Element zu verwenden.

Vorteile der Erfindung

Üblicherweise müssen in Systemen zur Überwachung von Kühl-Schmierstoffemulsionen die Sensoren zur Kalibrierung ausgebaut werden oder es muss dem System eine von der Kühl-Schmierstoffemulsion verschiedene Kalibrierflüssigkeit zugeführt werden, mit der die Messgenauigkeit der Sensoren überprüft wird. Eine solche Kalibrierflüssigkeit muss in der Regel wieder aus dem System ausgeschleust und entsorgt werden, da diese die Eigenschaften der Kühl-Schmierstoffemulsion nachteilig beeinflussen kann.

Die gemäß dem optionalen Schritt i) des erfindungsgemäßen Verfahrens vorgeschlagene Kalibrierung unter Verwendung einer frischen Kühl-Schmierstoffemulsion stellt hingegen ein einfaches abfallfreies Verfahren dar, mit dem der Verschmutzungszustand der Sensoren jederzeit festgestellt werden kann. Des Weiteren wird gemäß dem optionalen Schritt i) des Verfahrens eine frische Kühl-Schmierstoffemulsion mit definierten Eigenschaften bereitgestellt, welche für eine Kalibrierung des mindestens einen Sensors verwendet werden kann. Auch hier wird vorteilhaft der Einsatz von Referenzemulsionen oder Kalibrierflüssigkeiten vermieden, so dass keine Abfälle entstehen.

Die gemäß dem erfindungsgemäßen Verfahren vorgeschlagene Messung in einem Kreislauf trägt ebenfalls dazu bei, Abfälle zu vermeiden. In bevorzugten Ausführungsformen werden hierbei Mess-Reagenzien eingesetzt, welche die Eigenschaften der Kühl-Schmierstoffemulsion nicht nachteilig beeinflussen und daher nach Abschluss des Messvorgangs in den Tank zurückgegeben werden können.

Die Vorgeschlagene Vorrichtung ermöglicht eine einfache und sichere Handhabung von Kühl-Schmierstoffemulsionen, wobei der Zustand einer in einem Tank aufgenommenen Kühl-Schmierstoffemulsion überwacht werden kann und durch das Zugeben von Additiven bei Bedarf eine Konditionierung erfolgen kann. Vorteilhafterweise laufen dabei die Messung und die Konditionierung automatisiert ab.

Kurze Beschreibung der Figuren

Ausführungsbeispiele der Erfindung sind in den Zeichnungen dargestellt und in der nachfolgenden Beschreibung näher erläutert. Es zeigen:

Figur 1 eine schematische Darstellung einer Ausführungsform einer Vorrichtung zur Messung eines Parameters von Kühl-Schmierstoffemulsionen,

Figur 2 einen Ausschnitt der Vorrichtung der Figur 1, in dem eine Messeinheit der Vorrichtung dargestellt ist,

Figur 3 eine schematische Darstellung einer Vorrichtung zur Messung eines Parameters von Kühl-Schmierstoffemulsionen, welche mit mehreren Tanks verbunden ist,

Figur 4 eine Schnittansicht eines Refraktometers, und

Figur 5 schematisch das Messen des Füllstands in einem Konzentratvorratsbehälter.

In der nachfolgenden Beschreibung der Ausführungsbeispiele der Erfindung werden gleiche oder ähnliche Komponenten mit gleichen Bezugszeichen bezeichnet, wobei auf eine wiederholte Beschreibung dieser in Einzelfällen verzichtet wird. Die Figuren stellen den Gegenstand der Erfindung nur schematisch dar.

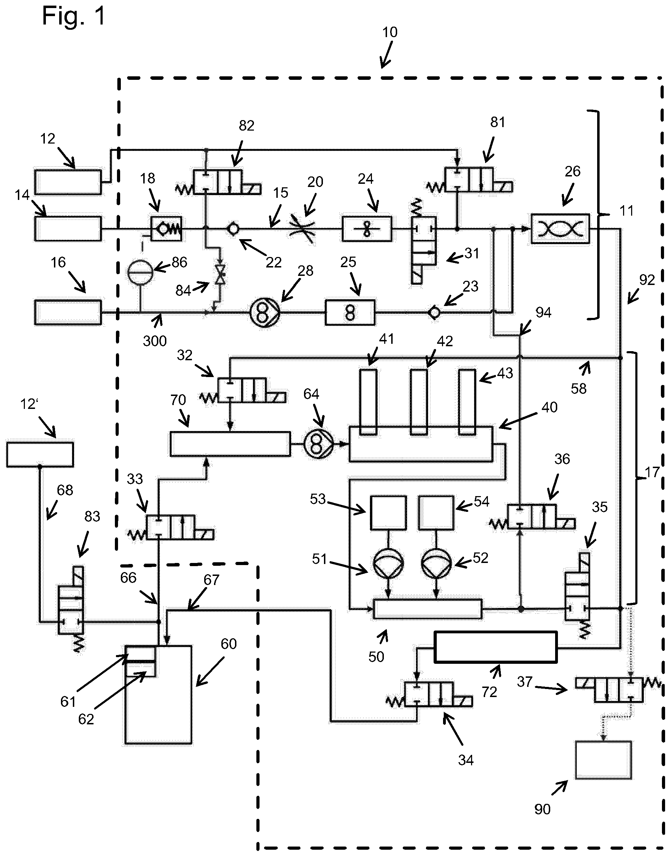

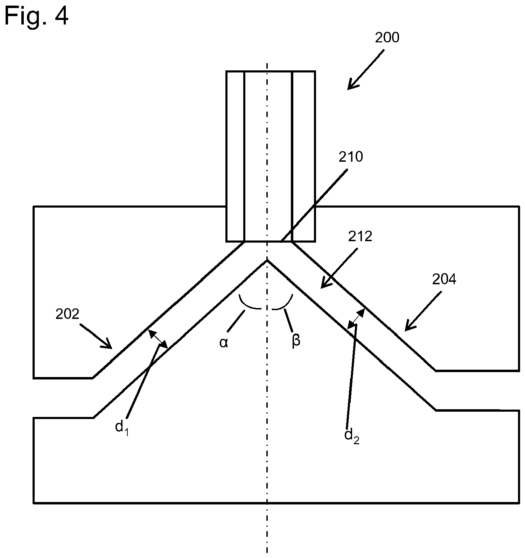

Figur 1 zeigt eine schematische Darstellung einer Ausführungsform einer Vorrichtung 10 zur Messung eines Parameters von Kühl-Schmierstoffemulsionen und zur Konditionierung von Kühl-Schmierstoffemulsionen. Die Vorrichtung 10 ist über eine Ansaugleitung 66 und eine Rückführleitung 67 mit einem Tank 60 verbunden, in welchem Kühl-Schmierstoffemulsion bevorratet wird. Aus dem Tank 60 werden eine oder mehrere Werkzeugmaschinen 110, 110', vergleiche Figur 4, mit der Kühl-Schmierstoffemulsion versorgt. Zur Überwachung des Füllpegels sind in dem dargestellten Ausführungsbeispiel zwei Füllstandsensoren 61, 62 im Tank 60 angeordnet. Beispielsweise ist ein erster Füllstandsensor 61 als ein Schwimmer ausgestaltet, der je nach Füllstand im Tank 60 ansteigt oder absinkt. Ein zweiter Füllstandsensor 62 ist als Ultraschallsensor ausgestaltet und erkennt auch Schaum, welcher gegebenenfalls auf der Kühl-Schmierstoffemulsion im Tank 60 liegt. In weiteren Ausführungsformen kann auch lediglich ein Sensor eingesetzt werden und es können auch andere Arten von Füllstandsensoren verwendet werden.

Die Vorrichtung 10 umfasst eine Bereitstellungseinheit 11, über die frische Kühl-Schmierstoffemulsion bereitgestellt werden kann. Des Weiteren umfasst die Vorrichtung 10 eine Messeinheit 17, über die mindestens ein Parameter der Kühl-Schmierstoffemulsion bestimmt werden kann.

Die Bereitstellungseinheit 11 umfasst eine Frischwasserleitung 15, welche mit einem Frischwasseranschluss 14 verbunden ist, und eine Konzentratansaugleitung 300, welche mit einem Konzentratbehälter 16 verbunden ist. Über den Frischwasseranschluss 14 wird Wasser bereitgestellt, bei dem es sich beispielsweise um Leitungswasser oder um aufbereitetes Wasser wie beispielsweise entmineralisiertes Wasser oder deionisiertes Wasser handeln kann. Der Konzentratbehälter 16 bevorratet ein Konzentrat, aus dem bei Verdünnung und Vermischung mit Wasser die gebrauchsfertige Kühl-Schmierstoffemulsion erhalten wird.

Wasser gelangt aus dem Frischwasseranschluss 14 über die Frischwasserleitung 15 in die Bereitstellungseinheit 11 und wird in einem Mischer 26 mit Konzentrat vermischt. Der Zufluss von Wasser wird dabei über ein erstes Ventil 31 gesteuert, wobei der Wasserdruck über ein Regelventil 20 eingestellt wird. Des Weiteren sind in der Frischwasserleitung 15 ein Systemtrenner 18 und ein erstes Rückschlagventil 22 angeordnet. Über den Systemtrenner 18 wird sichergestellt, dass aus der Bereitstellungseinheit 11 keine Stoffe in den Frischwasseranschluss 14 gelangen können.

Konzentrat wird aus dem Konzentratbehälter 16 über eine Konzentratpumpe 28 gefördert und dem Mischer 26 zugeleitet.

Ein zweites Rückschlagventil 23, welches in der Konzentratansaugleitung 300 angeordnet ist, verhindert, dass Wasser oder Kühl-Schmierstoffemulsion zurück in den Konzentratbehälter 16 fließen kann. Im Mischer 26 wird das Konzentrat mit dem Wasser vermischt. Um dabei eine Kühl-Schmierstoffemulsion mit einer definierten Konzentration zu erhalten, sind zwei Durchflusssensoren 24, 25 vorgesehen. Ein erster Durchflusssensor 24 erfasst die Menge an Wasser, die zum Mischer 26 fließt, und ein zweiter Durchflusssensor 25 erfasst die Menge an Konzentrat, die zum Mischer 26 gefördert wird. Anhand der über die Durchflusssensoren 24, 25 bestimmten Mengen an Wasser und Konzentrat werden die Konzentratpumpe 28 und/oder das Regelventil 20 derart geregelt, dass die nach dem Mischen erhaltene Kühl-Schmierstoffemulsion eine vorgegebene Konzentration aufweist. Alternativ hierzu ist es beispielsweise denkbar, zum Fördern des Konzentrats und/oder von Wasser volumetrische Pumpen einzusetzen, deren Fördervolumen exakt vorgegeben werden kann.

Die Bereitstellungseinheit gemäß der in Figur 1 dargestellten Ausführungsform umfasst des Weiteren eine Verbindung zu einer Druckluftquelle 12. Die Druckluftquelle 12 ist über ein erstes Druckluftventil 81 an einer Stelle zwischen dem ersten Ventil 31 und dem Mischer 26 mit der Frischwasserleitung 15 verbunden. Nach Beenden der Zuführung von Wasser und Konzentrat kann über das Druckluftventil 81 Druckluft in den Mischer 26 eingebracht werden, um noch darin befindliche Reste der Kühl-Schmierstoffemulsion herauszudrücken. Dabei befindet sich das Ventil 31 in einer geschlossenen Stellung, und es wird kein Konzentrat über die Konzentratpumpe 28 gefördert.

In der in Figur 1 dargestellten Ausführungsform ist eine Messung des Konzentratfüllstands im Konzentratbehälter 16 vorgesehen. Dazu wird ein kleiner Druckluftstrom von der Druckluftquelle 12 über ein zweites Druckluftventil 82 in Verbindung mit einem regelbaren Nadelventil 84 in die Ansaugleitung 300 gedrückt und der Druck wird stetig mit einem Drucksensor 86 gemessen. Die Messung des Konzentratfüllstands wird nachfolgend mit Bezug zur Figur 6 beschrieben.

Die Vorrichtung 10 umfasst des Weiteren eine Messeinheit 17, mit der mindestens ein Parameter der Kühl-Schmierstoffemulsion gemessen werden kann. Die in Figur 1 dargestellte Messeinheit 17 ist des Weiteren eingerichtet, der Kühl-Schmierstoffemulsion in Abhängigkeit des mindestens einen Parameters ein oder mehrere Additive zuzugeben.

Die Messeinheit 17 umfasst einen Verbindungsblock 70, einen Sensorblock 40 und einen Einmischblock 50.

Der Verbindungsblock 70 steht über eine Ansaugleitung 66 mit dem Tank 60 in Verbindung, so dass Kühl-Schmierstoffemulsion aus dem Tank 60 entnommen und zum Verbindungsblock 70 gefördert werden kann. Der Verbindungsblock 70 steht über eine Förderpumpe 64 mit dem Sensorblock 40 in Verbindung. Der Sensorblock 40 ist mit dem Einmischblock 50 verbunden. Vom Einmischblock 50 aus führt eine Leitung zu einem Knotenpunkt, von dem eine Rückführleitung 67 abzweigt, über die Kühl-Schmierstoffemulsion zurück in den Tank 60 geleitet werden kann, und von dem eine Bypassleitung 58 abzweigt, über die Kühl-Schmierstoffemulsion vom Einmischblock 50 zurück zum Verbindungsblock 70 geführt werden kann. Optional kann die Messeinheit 17 mit einem Auffangbehälter 90 verbunden sein, wobei eine Verbindungsleitung zum Auffangbehälter 90 ebenfalls von dem Knotenpunkt abzweigt. Die Verbindung zum Auffangbehälter 90 wird über ein siebtes Ventil 37 gesteuert.

Der Sensorblock 40 weist in dem in Figur 1 dargestellten Ausführungsbeispiel drei Sensoren 41, 42, 43 auf. Beispielsweise kann ein erster Sensor 41 als ein Refraktometer ausgestaltet sein, ein zweiter Sensor 42 kann als ein pH-Wert Sensor ausgestaltet sein und ein dritter Sensor 43 kann als ein Leitfähigkeitssensor ausgestaltet sein. In weiteren Ausführungsformen können abweichend davon andere Sensortypen und/oder eine andere Anzahl von Sensoren 41, 42, 43 verwendet werden.

Der in Figur 1 dargestellte Einmischblock 50 ist zum Einmischen von zwei verschiedenen Additiven und/oder Mess-Reagenzien eingerichtet. Dazu weist der Einmischblock zwei Pumpen 51, 52 sowie zwei Additivtanks 53, 54 auf. Eine erste Pumpe 51 ist eingerichtet, aus einem ersten Additivtank 53 ein erstes Additiv oder eine erste Mess-Reagenz in den Einmischblock 50 zu fördern. Eine zweite Pumpe 52 ist eingerichtet, aus einem zweiten Additivtank 54 ein zweites Additiv oder eine zweite Mess-Reagenz in den Einmischblock 50 zu fördern.

Die Messeinheit 17 und die Bereitstellungseinheit 11 sind in der in Figur 1 dargestellten Ausführungsform über zwei Leitungen miteinander verbunden. Eine erste Leitung 92 verbindet den Ausgang des Mischers 26 mit der Bypassleitung 58 der Messeinheit 17. Eine zweite Leitung 94 verbindet den Einmischblock 50 der Messeinheit 17 mit dem Eingang des Mischers 26 der Bereitstellungseinheit 11. Über Ventile 32, 33, 34, 35 und 36 kann der Fluss der Kühl-Schmierstoffemulsion in der Messeinheit 17 gesteuert werden. Ein zweites Ventil 32 ist in der Bypassleitung 58 angeordnet. Ein drittes Ventil 33 ist in der Ansaugleitung 66 angeordnet und ein viertes Ventil 34 ist in der Rückführleitung 67 angeordnet. Ein fünftes Ventil 35 ist stromabwärts des Einmischblocks 50 und vor der Bypassleitung 58 und vor der Rückführleitung 67 angeordnet. Über ein sechstes Ventil 36, welches in der zweiten Leitung 94 angeordnet ist, kann Kühl-Schmierstoffemulsion zurück in die Bereitstellungseinheit 11 geführt werden, um die Kühl-Schmierstoffemulsion erneut dem Mischer 26 zuzuführen.

Um Kühl-Schmierstoffemulsion aus der Ansaugleitung 66 und der Messeinheit 17 auszublasen, ist die Ansaugleitung 66 über eine Druckluftleitung 68 mit einer weiteren Druckluftquelle 12' verbunden. In der Druckluftleitung 68 ist ein drittes Druckluftventil 83 angeordnet, über welches das Ausblasen von Kühl-Schmierstoffemulsion gesteuert werden kann.

Die Vorrichtung 10 kann mehrere Betriebszustände realisieren. In einem ersten Betriebszustand wird Kühl-Schmierstoffemulsion aus dem Tank 60 bearbeitet. Die Kühl-Schmierstoffemulsion im Tank 60 wird zur Versorgung von Werkzeugmaschinen verwendet, wobei verwendete Kühl-Schmierstoffemulsion in den Tank 60 zurückgeführt wird. Durch den Gebrauch der Kühl-Schmierstoffemulsion können sich deren Zusammensetzung und damit deren Eigenschaften verändern. Zur Messung mindestens eines Parameters der gebrauchten Kühl-Schmierstoffemulsion wird diese über die Ansaugleitung 66 durch die Förderpumpe 64 aus dem Tank 60 gefördert, wobei diese das dritte Ventil 33 und den Verbindungsblock 70 passiert, und dem Sensorblock 40 zugeführt wird.

Für eine Messung mindestens eines Parameters der Kühl-Schmierstoffemulsion kann dieser im ersten Betriebszustand der Vorrichtung 10 kontinuierlich aus dem Tank 60 gefördert und durch den Sensorblock 40 und den Einmischblock 50 geleitet werden, so dass sowohl eine Messung mindestens eines Parameters der Kühl-Schmierstoffemulsion, als auch eine Konditionierung der Kühl-Schmierstoffemulsion durch Zugabe von Additiven ermöglicht ist. Nach dem Passieren des Einmischblocks 50 wird die Kühl-Schmierstoffemulsion über die Rückführleitung 67 zurück in den Tank 60 geleitet. Das vierte Ventil 34 und das fünfte Ventil 35 sind dabei geöffnet und das in der Bypassleitung 58 angeordnete zweite Ventil 32 ist geschlossen.

Im erfindungsgemäßen zweiten Betriebszustand der Vorrichtung 10, dem Messmodus, wird zunächst wie mit Bezug zum ersten Betriebszustand beschrieben eine Probe der Kühl-Schmierstoffemulsion aus dem Tank 60 über die Ansaugleitung 66 durch die Förderpumpe 64 in die Messeinheit 17 gefördert. Abweichend vom ersten Betriebszustand wird die Kühl-Schmierstoffemulsion nach dem Passieren des Einmischblocks 50 nicht in den Tank 60 zurückgeleitet, sondern über die Bypassleitung 58 zurück zum Verbindungsblock 70 geführt, so dass ein Kreislauf entsteht. In diesem Kreislauf wird die entnommene Probe der Kühl-Schmierstoff solange umgewälzt, bis die Messung des abgeschlossen ist.

Für diese Kreislaufführung sind das zweite Ventil 32 und das fünfte Ventil 35 geöffnet und das dritte Ventil 33 ist geschlossen. Das vierte Ventil 34 wird bevorzugt geöffnet, so dass bei einer Volumenausdehnung Kühl-Schmierstoffemulsion in den Tank 60 zurückfließen kann. Alternativ dazu kann das vierte Ventil 34 geschlossen werden, falls zu diesem Zeitpunkt kein Rückfluss in den Tank 60 gewünscht ist.

In dem zweiten Betriebszustand kann insbesondere eine Titration durchgeführt werden. Dabei wird zu der Probe, welche innerhalb der Messeinheit 17 umgewälzt wird, unter Verwendung des Einmischblocks 50 kontinuierlich oder schrittweise mindestens ein Mess-Reagenz zugegeben und es wird mindestens ein Parameter kontinuierlich gemessen. Erreicht dieser Parameter einen vorgegebenen Schwellenwert wird anschließend zumindest ein Teil der Probe zurück in den Tank 60 gefördert. Wird eine starke Durchmischung der Probe der Kühl-Schmierstoffemulsion mit dem Mess-Reagenz gewünscht, so kann durch Schließen des fünften Ventils 35 und Öffnen des sechsten Ventils 36 die Probe vom Ausgang des Einmischblocks 50 über den Mischer 26 zurück in den Verbindungsblock 70 geleitet werden. Alternativ oder zusätzlich ist es denkbar, stromabwärts des Einmischblocks 50 einen oder mehrere Agitatoren für eine Durchmischung oder eine Reaktion von Mess-Reagenzien mit der Probe anzuordnen.

Durch die kontinuierliche oder schrittweise Zugabe eines Mess-Reagenz erhöht sich das Volumen der Probe, welche innerhalb der Messeinheit 17 umgewälzt wird. Dies wird dadurch ausgeglichen, dass ein Teil der Probe kontinuierlich oder schrittweise entnommen wird. Die Entnahme eines Teils der Probe aus dem Kreislauf wird derart gesteuert, dass das Volumen der Probe innerhalb des Kreislaufs konstant gehalten wird. Der aus dem Kreislauf entnommene Teil der Probe kann in den Tank 60 und/oder in den Auffangbehälter 90 geleitet werden. Sofern die Probe nach dem Beenden der Messung nicht vollständig in den Tank 60 zurück gefördert wird, wird der verbleibende Teil der Probe in den Auffangbehälter 90 geleitet. Um das Entstehen von Abfällen zu vermeiden, ist es jedoch bevorzugt, die Probe vollständig in den Tank 60 zurück zu leiten.

In einem dritten Betriebszustand der Vorrichtung 10, dem Kalibriermodus, wird frische Kühl-Schmierstoffemulsion unter Verwendung der Bereitstellungseinheit 11 aus Wasser und Konzentrat hergestellt und zum Spülen und/oder Kalibrieren des Sensorblocks 40 der Messeinheit 17 verwendet. Die durch die Bereitstellungseinheit 11 hergestellte frische Kühl-Schmierstoffemulsion weist eine bekannte Konzentration und damit definierte Eigenschaften auf, welche für eine Kalibrierung der Sensoren 41, 42, 43 des Sensorblocks 40 genutzt werden. Die frische Kühl-Schmierstoffemulsion wird dazu ausgehend vom Mischer 26 über die Bypassleitung 58 zum Verbindungsblock 70 geführt und von dort aus über die Förderpumpe 64 durch den Sensorblock 40 geleitet. Von dort aus gelangt die frische Kühl-Schmierstoffemulsion über den Einmischblock 50 zum Verteilblock 72 und von dort aus in den Tank 60. Dementsprechend sind in dem dritten Betriebszustand das dritte Ventil 33, das sechste Ventil 36 und, sofern vorhanden, dass siebte Ventil 37 geschlossen. Das erste Ventil 31, das zweite Ventil 32, das vierte Ventil 34 und das fünfte Ventil 35 sind dabei geöffnet.

Zur Kalibrierung der Sensoren 41, 42, 43 des Sensorblocks 40 wird zunächst der Sensorblock 40 für eine vorgegebene Zeitspanne mit der frischen Kühl-Schmierstoffemulsion gespült, um eventuell vorhandene Verunreinigungen sowie Proben bereits verwendeter Kühl-Schmierstoffemulsion aus dem Sensorblock 40 zu entfernen. Anschließend werden mit den Sensoren 41, 42, 43 Messungen von Parametern der Kühl-Schmierstoffemulsion durchgeführt und mit Referenzwerten für die frische Kühl-Schmierstoffemulsion mit der bekannten Konzentration verglichen. Bei Abweichungen kann entweder eine Nachkalibrierung eines Sensors 41, 42 43 erfolgen oder es kann eine Aufforderung zu einer manuellen Reinigung oder eines Wechsels eines Sensors 41, 42, 43 erfolgen, sofern die Abweichungen nicht durch eine Veränderung der Kalibrierung ausgeglichen werden können.

Sowohl im ersten als auch im zweiten Betriebszustand der Vorrichtung 10 wird jeweils mindestens ein Parameter der im Tank 60 aufgenommenen Kühl-Schmierstoffemulsion bestimmt. Bevorzugt wird der mindestens eine Parameter mit einem Soll-Wert verglichen. Wird eine Abweichung festgestellt können Gegenmaßnahmen eingeleitet werden.

Bevorzugt wird bei Erkennen einer Abweichung eines Parameters der Kühl-Schmierstoffemulsion eine Konditionierung des Kühl-Schmierstoffs vorgenommen, indem der Kühl-Schmierstoffemulsion mindestens ein Additiv zugegeben wird. Hierzu wird wie mit Bezug zum ersten Betriebszustand der Vorrichtung 10 beschrieben kontinuierlich Kühl-Schmierstoffemulsion aus dem Tank 60 gefördert und durch den Sensorblock 40 und den Einmischblock 50 geleitet. Beim Passieren des Einmischblocks 50 wird der Kühl-Schmierstoffemulsion mindestens ein Additiv zugegeben, wobei die Art und Menge des Additivs bevorzugt in Abhängigkeit der ermittelten Abweichung bestimmt werden. Über die Rückführleitung 67 wird die auf diese Weise konditionierte Kühl-Schmierstoffemulsion zurück in den Tank 60 geleitet. Das vierte Ventil 34 und das fünfte Ventil 35 sind dabei geöffnet, dass in der Bypassleitung 58 angeordnete zweite Ventil 32 ist geschlossen.

Alternativ oder zusätzlich kann die im Tank 60 aufgenommene Kühl-Schmierstoffemulsion durch Zugabe von frischer Kühl-Schmierstoffemulsion konditioniert bzw. nachgefahren werden, wobei das Mischungsverhältnis von Konzentrat und Wasser der frischen Kühl-Schmierstoffemulsion bevorzugt in Abhängigkeit der ermittelten Abweichung insbesondere bei der Konzentration der Kühl-Schmierstoffemulsion bestimmt wird. Wurde über den mindestens einen Parameter beispielsweise ermittelt, dass durch Verdunstung von Wasser die Konzentration der Kühl-Schmierstoffemulsion im Tank 60 zu groß ist, also oberhalb eines vorgegebenen Grenzwerts liegt, so wird eine entsprechende Menge an frischer Kühl-Schmierstoffemulsion mit geringerer Konzentration und damit einem höheren Anteil an Wasser zugegeben.

Alternativ oder zusätzlich ist es möglich, die Konzentration der Kühl-Schmierstoffemulsion zu erhöhen, beispielsweise wenn aufgrund eines Wechsels des Bearbeitungsprozesses eine andere Konzentration der Kühl-Schmierstoffemulsion benötigt wird oder die Konzentration aus einem anderen Grund zu niedrig sein sollte. Hierzu ist vorgesehen, dass zweite Ventil 32 und das fünfte Ventil 35 zu schließen und das dritte Ventil 33, das vierte Ventil 34 und das sechste Ventil 36 zu öffnen. Kühl-Schmierstoffemulsion wird dann aus dem Tank 60 über die Förderpumpe 64 durch den Sensorblock 40 und den Einmischblock 50 zum Mischer 26 geleitet. Des Weiteren wird über die Konzentratpumpe 28 Konzentrat aus dem Konzentratbehälter 16 gefördert und ebenfalls dem Mischer 26 zugeführt. Im Mischer 26 wir dann die Kühl-Schmierstoffemulsion aus dem Tank 60 mit dem Konzentrat aus dem Konzentratbehälter 16 vermischt und anschließend über den Verteilblock 72 wieder in den Tank 60 geleitet. Auf diese Weise kann die Konzentration des Kühl-Schmierstoffs im Tank 60 auch dann erhöht werden, wenn der Füllstand im Tank 60 bereits sehr hoch ist und daher kein Nachfahren mit frischer Kühl-Schmierstoffemulsion höherer Konzentration möglich wäre.

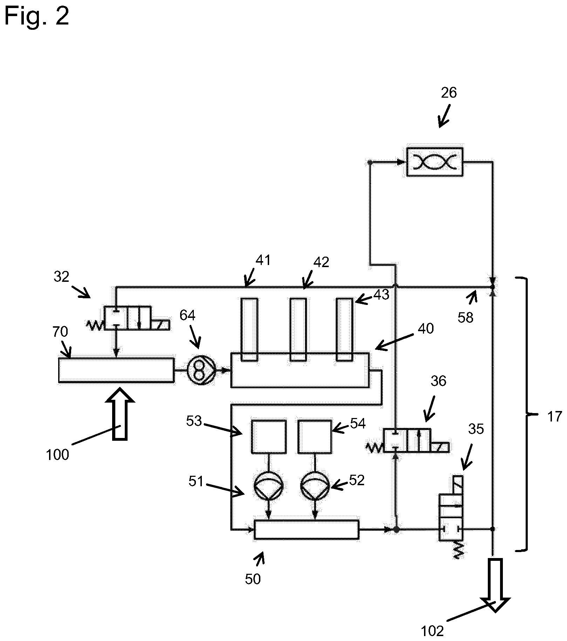

Figur 2 zeigt einen Ausschnitt der Vorrichtung der Figur 1, in dem eine Messeinheit 17 der Vorrichtung 10 dargestellt ist. Die Messeinheit 17 entspricht dabei der mit Bezug zur Figur 1 beschriebenen Messeinheit 17. Der mit dem Bezugszeichen 100 versehene Pfeil markiert eine Zugabe von Kühl-Schmierstoffemulsion in die Messeinheit 17 aus einem Tank 60, vergleiche Figur 1. Der mit dem Bezugszeichen 102 versehene Pfeil markiert eine Entnahme von Kühl-Schmierstoffemulsion aus der Messeinheit 17.

Im zweiten Betriebszustand der Vorrichtung 10, dem Messmodus, wird eine zu untersuchende Probe der Kühl-Schmierstoffemulsion wie mit dem Pfeil 100 markiert in die Messeinheit 17 bzw. in den Verbindungsblock 70 eingegeben. Von dort aus wird Kühl-Schmierstoffemulsion über die Förderpumpe 64 durch den Sensorblock 40 und den Einmischblock 50 geleitet. In einer Variante des zweiten Betriebszustands ist das sechste Ventil 36 geschlossen und das fünfte Ventil 35 und das zweite Ventil 32 sind geöffnet, so dass Kühl-Schmierstoffemulsion nach Passieren des Einmischblocks 50 über die Bypassleitung 58 und das zweite Ventil 32 direkt zurück in den Verbindungsblock 70 geleitet wird. In einer zweiten Variante des zweiten Betriebszustands ist abweichend davon das sechste Ventil 36 geöffnet und das fünfte Ventil 35 geschlossen, so dass die Kühl-Schmierstoffemulsion nach dem Passieren des Einmischblocks 50 zunächst durch den Mischer 26 geleitet wird und erst nach Passieren des Mischers 26 über die Bypassleitung 58 zurück in den Verbindungsblock 70 gelangt.

Um bei einer Messung, bei der im Einmischblock 50 eine oder mehrere Mess-Reagenzien zugegeben werden, das Volumen der im Kreislauf geführten Kühl-Schmierstoffemulsion konstant zu halten, kann entsprechend des zugegebenen Volumens an Mess-Reagenzien ein Teil der im Kreislauf geführten Kühl-Schmierstoffemulsion an der mit dem Pfeil 102 markierten Stelle entnommen werden.

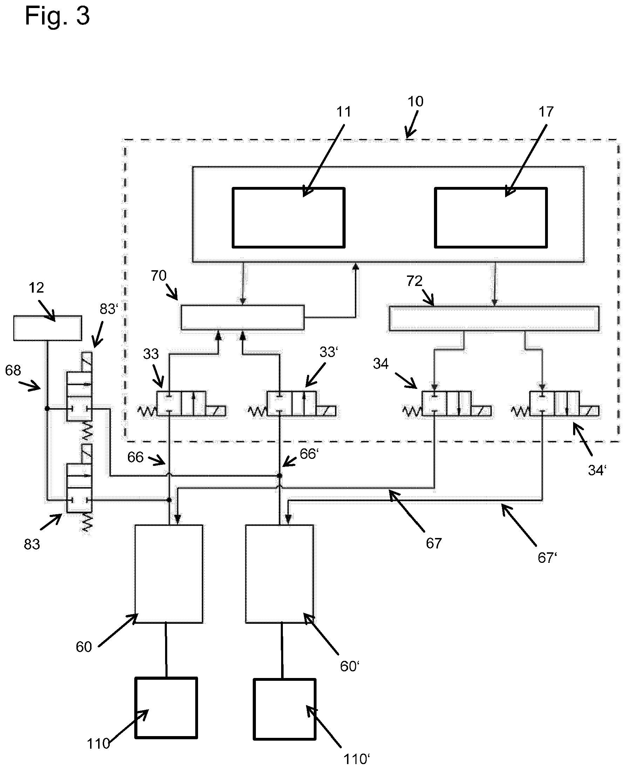

Figur 3 zeigt eine schematische Darstellung einer Vorrichtung 10 zur Messung eines Parameters und zur Konditionierung von Kühl-Schmierstoffemulsionen, welche mit mehreren Tanks 60, 60' verbunden ist. In dem in der Figur 3 dargestellten Beispiel ist die Vorrichtung mit zwei Tanks 60, 60' verbunden. Für eine Entnahme von Kühl-Schmierstoffemulsion aus den Tanks 60, 60' ist der Verbindungsblock 70 der Vorrichtung 10 über die Ansaugleitung 66 und das dritte Ventil 33 mit dem Tank 60 verbunden und über eine weitere Ansaugleitung 66' und einem weiteren dritten Ventil 33' mit dem weiteren Tank 60' verbunden.

Zum Einleiten von Kühl-Schmierstoffemulsion in die beiden Tanks 60, 60' ist der Verteilblock 72 der Vorrichtung 10 über das vierte Ventil 34 und die Rückführleitung 67 mit dem Tank 60 und über ein weiteres viertes Ventil 34' und eine weitere Rückführleitung 67' mit dem weiteren Tank 60' verbunden.

Die Vorrichtung 10 ist bevorzugt immer nur mit einem der Tanks 60, 60' zur gleichen Zeit verbunden. Bei Verbindung mit dem Tank 60 ist somit vorgesehen, dass das weitere dritte Ventil 33' und das weitere vierte Ventil 34' geschlossen sind. Je nach Betriebszustand der Vorrichtung 10 werden dann das dritte Ventil 33 und das vierte Ventil 34 geöffnet. Bei Verbindung der Vorrichtung 10 mit dem weiteren Tank 60' sind umgekehrt das dritte Ventil 33 und das vierte Ventil 34 geschlossen, während je nach Betriebszustand der Vorrichtung 10 das weitere dritte Ventil 33' und das weitere vierte Ventil 34' geöffnet sind.

Um Kühl-Schmierstoffemulsion aus der Ansaugleitung 66 und der Messeinheit 17 der Vorrichtung 10 auszublasen, ist die Ansaugleitung 66 über die Druckluftleitung 68 und das dritte Druckluftventil 83 mit der Druckluftquelle 12 verbunden. Des Weiteren ist die Druckluftquelle 12 über ein weiteres drittes Druckluftventil 83' mit der weiteren Ansaugleitung 66' verbunden.

In weiteren Ausführungsvarianten kann die Anzahl der Tanks 60, 60', welche mit der Vorrichtung 10 verbunden werden, variiert werden. Beispielsweise werden im Bereich von zwei bis zehn Tanks 60, 60' verwendet, wobei deren Ansaugleitungen 66, 66' jeweils über ein drittes Ventil 33 mit dem Verbindungsblock 70 und deren Rückführleitungen 67, 67' jeweils über ein viertes Ventil 34 mit dem Verteilblock 72 verbunden werden.

Figur 4 zeigt eine Schnittansicht eines Refraktometers 200, welches als ein Sensor 41, 42, 43 eingesetzt werden kann. Das Refraktometer 200 weist ein Messfenster 210 auf, welches einen optischen Zugang zu einer in einem Kanal 212 geführten Flüssigkeit ermöglicht. Bei der Flüssigkeit handelt es sich bevorzugt um eine zu untersuchende Kühl-Schmierstoffemulsion.

Die zu untersuchende Flüssigkeit wird über einen Zulauf 202 zum Messfenster 210 geleitet und strömt dabei das Messfenster 210 unter einem Winkel $\alpha$ an. Entsprechend weist der Zulauf 202 zu einer Senkrechten zum Messfenster 210 den Winkel $\alpha$ auf. Nach Passieren des Messfensters 210 führt ein Ablauf 204 die Flüssigkeit ab. Der Ablauf 204 ist in einem Winkel $\beta$ zur Senkrechten zum Messfenster 210 angeordnet. In der Darstellung der Figur 4 sind die beiden Winkel $\alpha$, $\beta$ gleich groß gewählt. In einer weiteren Ausführungsform können die Winkel $\alpha$, $\beta$ aber auch unterschiedlich

groß gewählt werden.

Der Zulauf 202 weist dabei einen ersten Durchmesser d1 und der Ablauf 204 einen zweiten Durchmesser d2 auf, wobei in dem in Figur 4 dargestellten Beispiel die Durchmesser des Zulaufs 202 und des Ablaufs 204 identisch gewählt sind. In weiteren Ausführungsformen können die Durchmesser von Zulauf 202 und Ablauf 204 auch unterschiedlich gewählt werden.

Bevorzugt werden die Durchmesser d1 und d2 auf einen vorgegebenen Durchfluss der Flüssigkeit durch das Refraktometer 200 ausgelegt, wobei bei dem vorgegebenen Durchfluss eine vorgegebene Strömungsgeschwindigkeit der Flüssigkeit am Messfenster 210 erzielt wird. Für den Fall, dass die Durchmesser im Zulauf 202 und Ablauf 204 identisch sind ist die Strömungsgeschwindigkeit v gegeben durch

$$v = V/F$$

wobei V der Durchfluss der Flüssigkeit ist und F die durchströmte Fläche ist, welche bei einem Kanal 212 mit rundem Querschnitt über den Durchmesser leicht berechnet werden kann.

Bevorzugt werden der erste und der zweite Durchmesser derart gewählt, dass sich bei dem vorgegebenen Durchfluss am Messfenster 210 eine Strömungsgeschwindigkeit im Bereich von 1 m/min bis 500 m/min einstellt.

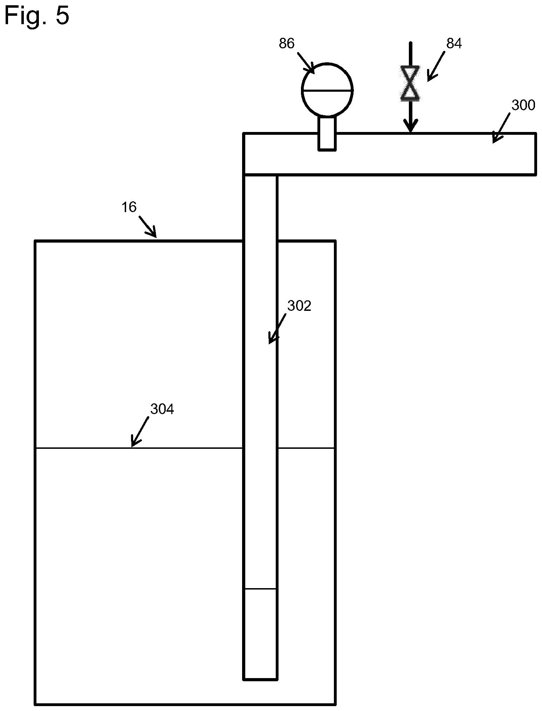

Figur 5 zeigt schematisch das Messen eines Füllpegels 304 im Konzentratbehälter 16 unter Verwendung der mit Bezug zur Figur 1 beschriebenen Vorrichtung 10. In der Figur 5 ist die Konzentratansaugleitung 300 der Vorrichtung 10 dargestellt. In der Konzentratansaugleitung 300 ist der Drucksensor 86 angeordnet und über das Nadelventil 84 kann gezielt eine kontrollierte Menge an Druckluft in die Konzentratansaugleitung 300 eingeleitet werden. Die Konzentratansaugleitung 300 mündet in ein Steigrohr 302, welches in den Konzentratbehälter 16 eingeführt ist und sich unterhalb des Füllpegels 304 in den Konzentratbehälter 16 öffnet.

Zur Messung des Füllpegels 304 des Konzentrats im Konzentratbehälter 16 wird ein kleiner Druckluftstrom von der Druckluftquelle 12 über das zweite Druckluftventil 82, vergleiche Figur 1, in Verbindung mit dem regelbaren Nadelventil 84 in die Ansaugleitung 300 gedrückt. Dieser Druckluftstrom drückt das Konzentrat im Konzentratbehälter 16 langsam aus dem Steigrohr 302, was zu einem langsamen Druckanstieg gemäß dem hydrostatischen Druck führt. Dieser Druck wird stetig mit einem Drucksensor 86 gemessen. Sobald die Druckluft aus dem Steigrohr 302 im Konzentratbehälter 16 tritt, steigt der Druck nicht weiter an und aus dem dann gemessenen Grenzdruck P lässt sich mittels der Formel

$$P = \rho \cdot g \cdot h$$

der Füllstand im KSS-Behälter berechnen, wobei $\rho$ die Dichte des Konzentrats ist, g die Erdbeschleunigung ist und h die Höhe des Flüssigkeitsspiegels oberhalb der Öffnung des Steigrohrs ist.

Beispiele

In den nachfolgenden Beispielen wurden verschiedene Kühl-Schmierstoffemulsionen verwendet:
Bei der Kühl-Schmierstoffemulsion KSS1 handelt es sich um eine Emulsion, welche durch Verdünnen eines Konzentrats erhalten wird, welches unter der Bezeichnung Blasocut 4000 Strong von Blaser Swisslube erhältlich ist. Blasocut 4000 Strong ist ein wassermischbarer Kühlschmierstoff auf Mineralölbasis.

Bei der Kühl-Schmierstoffemulsion KSS2 handelt es sich um eine Emulsion, welche durch Verdünnen eines Konzentrats erhalten wird, welches unter der Bezeichnung Vasco 7000 von Blaser Swisslube erhältlich ist. Vasco 7000 ist ein wassermischbarer Kühlschmierstoff auf Esterölbasis.

Bei der Kühl-Schmierstoffemulsion KSS3 handelt es sich um eine Emulsion, welche durch Verdünnen eines Konzentrats erhalten wird, welches unter der Bezeichnung B-Cool 755 von Blaser Swisslube erhältlich ist. B-Cool 755 ist ein wassermischbarer Kühlschmierstoff auf Mineralölbasis.

Titration zur Messung der Pufferkapazität

Die Messung der Pufferkapazität erfolgt durch Messung der Menge an Säure, welche zum Erreichen eines bestimmten pH-Wertes notwendig ist. Üblicherweise wird zur Messung der Pufferkapazität eine Probe der Kühlschmierstoffemulsion entnommen und im Labor durch Säure-Base Titration gemessen. Die Bestimmung der Pufferkapazität wurde für zwei verschiedene Kühl-Schmierstoffemulsionen durchgeführt, einmal mit der erfindungsgemäßen Vorrichtung und zum Vergleich im Labor.

Die Konzentration der ersten Kühl-Schmierstoffemulsion KSS1 betrug bei dem Versuch 10%. Bei der zweiten Kühl-Schmierstoffemulsion KSS2 wurde eine Konzentration von 10% verwendet.

Versuch, Untersuchung in erfindungsgemäßer Vorrichtung

Die untersuchten Kühl-Schmierstoffemulsionen wurden in eine Vorrichtung 10 wie mit Bezug zur Figur 1 beschrieben eingeleitet, wobei der optionale Auffangbehälter 90 nicht verwendet wurde. Die Vorrichtung 10 wurde derart betrieben, dass die Förderpumpe 64eingeschaltet war. Das dritte Ventil 33 war geschlossen, das zweite Ventil 32, das vierte Ventil 34 und das fünfte Ventil 35 waren geöffnet.

Essigsäure wurde im ersten Additivtank 53 bevorratet und über die erste Pumpe 51 zudosiert. Dabei wurde die Essigsäure jeweils in kleinen Schritten zudosiert und anschließend abgewartet, bis sich die Essigsäure homogen in der Emulsion verteilt hat. Anschließend wurde der pH-Wert gemessen. Das Zudosieren und das Messen wurden wiederholt, bis ein vordefinierter pH-Wert von 7 erreicht wurde. Anschließend wurde das Gesamtvolumen der zudosierten Säure bis zum Erreichen des vordefinierten pH-Werts bestimmt. Die Ergebnisse sind in Tabelle 1 aufgelistet.

(Tabelle 1)

|  | Verbrauch in ml | Relative Pufferkapazität |
|---|---|---|
| KSS 1, 10%ig | 23 | 3,1 |
| KSS 2, 10%ig | 7,5 | 1 |

Vergleichsversuch, Untersuchung im Labor:

Es wurden Proben der Kühl-Schmierstoffemulsionen entnommen und im Labor untersucht. Das Volumen der Probe entspricht dem Volumen, welches auch durch die erfindungsgemäße Vorrichtung behandelt wurde. Die Ergebnisse sind in Tabelle 2 dargestellt.

(Tabelle 2)

|  | Verbrauch in ml | Relative Pufferkapazität |
|---|---|---|
| KSS 1, 10%ig | 22,8 | 3 |
| KSS 2, 10%ig | 7,6 | 1 |

Die automatisiert durchgeführte Titration liefert im Rahmen der Messtoleranzen das gleiche Ergebnis wie die zum Vergleich ausgeführte Messung im Labor.

Erstbefüllung und Nachfahren des Tanks

Unter Verwendung der mit Bezug zu den Figuren 1 und 3 beschriebenen erfindungsgemäßen Vorrichtung 10 wird für eine Erstbefüllung von zwei Tanks sowie später für das Nachfahren der Tanks frische Kühl-Schmierstoffemulsion jeweils mit einer vorgegebenen Konzentration bereitgestellt. Die Vorrichtung 10 ist dazu mit zwei verschiedenen Tanks 60, 60' verbunden, welche beide unter Verwendung der Vorrichtung 10 bearbeitet werden.

Für eine Erstbefüllung wird dazu über die Bereitstellungseinheit 11 der Vorrichtung 10 die Kühl-Schmierstoffemulsion in der gewünschten Konzentration bereitgestellt und in den Tank 60, 60' eingeleitet. Für das Nachfahren wird zunächst unter Verwendung der Messeinheit 17 der Vorrichtung 10 die Konzentration gemessen.

Des Weiteren wird über Füllstandssensoren 61,62 der Füllstand in den Tanks 60, 60' gemessen und in Abhängigkeit der gemessenen Konzentration und des Füllstands eine Soll-Konzentration für die für das Nachfahren bereitgestellte frische Kühl-Schmierstoffemulsion bestimmt. Anschließend wird frische Kühl-Schmierstoffemulsion mit dieser Konzentration bereitgestellt und in den jeweiligen Tank 60, 60' geleitet.

Versuch 1

Unter Verwendung der erfindungsgemäßen Vorrichtung wird ein Tank mit der Kühl-Schmierstoffemulsion KSS1 nachgefahren. Für den Tank 1 ist eine Konzentration von 6% vorgegeben und für den Tank 2 ist eine Konzentration von 8 % vorgegeben. In der Tabelle 1 sind die vor dem Nachfahren gemessenen Füllstände und Konzentrationen aufgelistet. Des Weiteren ist in Tabelle 3 angegeben, welche Soll-Konzentration für die bereitgestellte frische Kühl-Schmierstoffemulsion bestimmt wurde und welche tatsächliche Konzentration für die frische Kühl-Schmierstoffemulsion gemessen wurde. In der Tabelle 4 sind die nach dem Nachfahren in den Tanks ermittelten Füllstände und Konzentrationen aufgelistet.

(Tabelle 3)

| Tank | Vorher | | | | | |
| --- | --- | --- | --- | --- | --- | --- |
| | Füllstand [l] | | Konzentration [%] | | Nachfahrkonzentration [%] | |
| | ist | soll | ist | soll | berechnet | gemessen |
| 1 | 58,5 | 80,0 | 5,9 | 6,0 | 6,4 | 6,5 |
| 2 | 41,0 | 80,0 | 8,2 | 8,0 | 8,3 | 8,2 |

(Tabelle 4)

| Tank | Nachher | |
| --- | --- | --- |
| | Füllstand [l] | Konzentration [%] |
| | Ist | ist |
| 1 | 81,5 | 6,2 |
| 2 | 81,9 | 8,1 |

Versuch 2

Unter Verwendung der erfindungsgemäßen Vorrichtung wird ein Tank mit der Kühl-Schmierstoffemulsion KSS3 zunächst erstmals befüllt. Später wird der Tank nachgefahren. Für den Tank ist eine Konzentration von 5% vorgegeben In der Tabelle 5 sind die vor dem Nachfahren bzw. Erstbefüllen gemessenen Füllstände und Konzentrationen aufgelistet. Des Weiteren ist in Tabelle 5 angegeben, welche Soll-Konzentration für die bereitgestellte frische Kühl-Schmierstoffemulsion bestimmt wurde und welche tatsächliche Konzentration für die frische Kühl-Schmierstoffemulsion gemessen wurde. In der Tabelle 6 sind die nach dem Nachfahren bzw. Befüllen ermittelten Füllstände und Konzentrationen aufgelistet.

(Tabelle 5)

| Operation | Vorher | | | | | |
| --- | --- | --- | --- | --- | --- | --- |
| | Füllstand [l] | | Konzentration [%] | | Nachfahrkonzentration [%] | |
| | ist | soll | ist | soll | berechnet | Gemessen |
| Erstbefüllung | 0,0 | 50,0 | 0,0 | 5,0 | 5,0 | 5,1 |
| Automatisches Nachfahren | 49,5 | 70,0 | 5,1 | 5,0 | 4,8 | 4,9 |

(Tabelle 6)

| Operation | Nachher | |
| --- | --- | --- |
| | Füllstand [l] | Konzentration [%] |
| | ist | ist |
| Erstbefüllung | 49,5 | 5,1 |
| Automatisches Nachfahren | 72,0 | 5,0 |

Die Erfindung ist nicht auf die hier beschriebenen Ausführungsbeispiele und die darin hervorgehobenen Aspekte beschränkt. Vielmehr ist innerhalb des durch die Ansprüche angegebenen Bereichs eine Vielzahl von Abwandlungen möglich, die im Rahmen fachmännischen Handelns liegen.

Bezugszeichenliste

| | |
|---|---|
| 10 | Vorrichtung |
| 11 | Bereitstellungseinheit |
| 12 | Druckluftquelle |
| 14 | Frischwasseranschluss |
| 15 | Frischwasserleitung |
| 16 | Konzentratbehälter |
| 17 | Messeinheit |
| 18 | Systemtrenner |
| 20 | Druckregelventil |
| 22 | 1. Rückschlagventil |
| 23 | 2. Rückschlagventil |
| 24 | 1. Durchflusssensor |
| 25 | 2. Durchflusssensor |
| 26 | Mischer |
| 28 | Konzentratpumpe |
| 31 bis 37 | Ventil |

| | |
|---|---|
| 40 | Sensorblock |
| 41 | 1. Sensor |
| 42 | 2. Sensor |
| 42 | 3. Sensor |

| | |
|---|---|
| 50 | Einmischblock |
| 51 | 1. Additivpumpe |
| 52 | 2. Additivpumpe |
| 53 | 1. Additivtank |
| 54 | 2. Additivtank |

| | |
|---|---|
| 58 | Bypass-Leitung |
| 60 | Tank |
| 60' | weiterer Tank |
| 61 | 1. Füllstandssensor |
| 62 | 2. Füllstandssensor |
| 64 | Förderpumpe |
| 66 | Ansaugleitung |
| 68 | Druckluftleitung |
| 70 | Verbindungsblock |
| 72 | Verteilblock |

| | |
|---|---|
| 81 | 1. Druckluftventil |
| 82 | 2. Druckluftventil |
| 83 | 3. Druckluftventil |
| 84 | Nadelventil |
| 86 | Drucksensor |

| | |
|---|---|
| 90 | Auffangbehälter |
| 92 | 1. Leitung |
| 94 | 2. Leitung |

| | |
|---|---|
| 100 | Zugabe |
| 102 | Entnahme |
| 110 | Werkzeugmaschine |

| | |
|---|---|
| 200 | Refraktometer |

| 202 | Zulaufleitung |
|---|---|
| 204 | Ablaufleitung |
| 210 | Messfenster |
| 212 | Kanal |

| 300 | Konzentratansaugleitung |
|---|---|
| 302 | Steigrohr |
| 304 | Füllpegel |

**Patentansprüche**

1. Verfahren zur Bestimmung mindestens eines Parameters einer Kühl-Schmierstoffemulsion für Werkzeugmaschinen (110, 110'), wobei eine Kühl-Schmierstoffemulsion in einem Tank (60, 60') bereitgestellt wird und wobei die Kühl-Schmierstoffemulsion aus dem Tank (60, 60') entnommen wird, unter Verwendung mindestens eines Sensors (41, 42, 43) mindestens ein Parameter der Kühl-Schmierstoffemulsion erfasst wird und die Kühl-Schmierstoffemulsion zumindest teilweise in den Tank (60, 60') zurückgeführt wird, **dadurch gekennzeichnet, dass** das Verfahren zusätzlich das Durchführen einer Messung umfasst, bei der eine Probe aus dem Tank (60, 60') entnommen wird, die Probe in einem Kreislauf fortlaufend an dem mindestens einen Sensor (41, 42, 43) vorbeigeführt wird, um mindestens einen Parameter der Probe zu erfassen, wobei kontinuierlich oder schrittweise mindestens ein Mess-Reagenz zugegeben wird und der mindestens eine Parameter kontinuierlich gemessen wird, bis der mindestens eine Parameter einen vorgegebenen Schwellenwert erreicht, und anschließend zumindest ein Teil der Probe zurück in den Tank (60, 60') gefördert wird.

2. Verfahren gemäß Anspruch 1, **dadurch gekennzeichnet, dass** ein Teil der Probe als Abfall verworfen und nicht in den Tank (60, 60') zurückgeführt wird oder dass die Probe vollständig in den Tank (60, 60') zurückgeführt wird.

3. Verfahren gemäß Anspruch 1 oder 2, **dadurch gekennzeichnet, dass** unter Verwendung eines Agitators eine Vermischung und/oder eine chemische Reaktion zwischen der im Kreislauf geführten Kühl-Schmierstoffemulsion und dem Mess-Reagenz durchgeführt wird.

4. Verfahren gemäß einem der Ansprüche 1 bis 3, **dadurch gekennzeichnet, dass** vor dem kontinuierlichen Zugeben der Messreagenz mindestens eine diskontinuierliche Zugabe einer weiteren Messreagenz erfolgt.

5. Verfahren nach einem der Ansprüche 1 bis 4, **dadurch gekennzeichnet, dass** das Volumen der Probe bei Zugabe der Messreagenz und/oder der weiteren Messreagenz konstant gehalten wird, indem ein Teil der Probe aus dem Kreislauf entnommen und in den Tank (60, 60') zurückgeführt wird.

6. Verfahren gemäß Anspruch 4 oder 5, **dadurch gekennzeichnet, dass** eine Messung des Total-Amin / Total-Säure Wertes (TA/TS-Wert) durchgeführt wird, wobei das weitere Messreagenz eine Säure ist und eine Base als Messreagenz kontinuierlich zugegeben wird und wobei der mindestens eine Parameter der pH-Wert des Kühlschmierstoffs ist.

7. Verfahren gemäß einem der Ansprüche 1 bis 6 **dadurch gekennzeichnet, dass** eine Säure-Base-Titration zur Messung einer Pufferkapazität der Kühl-Schmierstoffemulsion durchgeführt wird, wobei das Mess-Reagenz eine Maßlösung ausgewählt aus einer Säure oder einer Lauge und der mindestens eine Parameter der pH-Wert ist oder dass ein Metallgehalt der Kühl-Schmierstoffemulsion bestimmt wird, wobei das Mess-Reagenz ausgewählt ist aus einem Komplexbildner und der mindestens eine Parameter eine Lichtdurchlässigkeit oder ein Reflexionsgrad der Kühl-Schmierstoffemulsion für Licht ist oder dass ein Ölgehalt der Kühl-Schmierstoffemulsion bestimmt wird, wobei das Mess-Reagenz ausgewählt ist aus einer konzentrierten Salzlösung oder eine Säure und über den mindestens einen Sensor der Anteil der sich bildenden Ölphase erkannt wird oder dass ein Nitritgehalt der Kühl-Schmierstoffemulsion bestimmt wird und der mindestens eine Parameter eine Durchlässigkeit der Kühl-Schmierstoffemulsion für Licht ist.

8. Verfahren gemäß einem der Ansprüche 1 bis 7, **dadurch gekennzeichnet, dass** frische Kühl-Schmierstoffemulsion durch Fördern eines Konzentrats aus einem Konzentratvorratsbehälter (16) und Mischen des Konzentrats mit Wasser bereitgestellt wird, wobei das Konzentrat über eine Konzentratansaugleitung (300) mit einem Steigrohr (302) aus dem Konzentratvorratsbehälter (16) gefördert wird und wobei ein Füllpegel (304) des Konzentrats im Konzentratvorratsbehälter (16) bestimmt wird durch Einleiten von Druckluft in die Konzentratansaugleitung (300), wobei ein Luftdruck in der Konzentratansaugleitung (300) mit einem Drucksensor (86) gemessen wird, ein Grenzdruck für einen Druckanstieg bestimmt wird und unter Verwendung des Grenzdrucks der Füllpegel (304) im Konzentratvorratsbehälter (16) berechnet wird.

9.  Verfahren gemäß einem der Ansprüche 1 bis 8, **dadurch gekennzeichnet, dass** die Kühl-Schmierstoffemulsion konditioniert wird, indem in Abhängigkeit des mindestens einen Parameters mindestens ein Additiv zur Kühl-Schmierstoffemulsion zugegeben wird und die konditionierte Kühl-Schmierstoffemulsion in den Tank (60, 60') zurückgeführt wird.

10. Verfahren gemäß Anspruch 9, **dadurch gekennzeichnet, dass** das Additiv unverdünntes Konzentrat zur Herstellung einer Kühl-Schmierstoffemulsion ist, wobei die Menge des Konzentrats in Abhängigkeit des mindestens einen Parameters bestimmt wird.

11. Vorrichtung (10) zur Bestimmung mindestens eines Parameters einer Kühl-Schmierstoffemulsion für Werkzeugmaschinen (110, 110') umfassend einen Sensorblock (40) umfassend mindestens einen Sensor (41, 42, 43), einen Einmischblock (50) umfassend wenigstens eine Additivpumpe (51, 52) zum Einmischen eines Additivs oder eines Mess-Reagenz und

    eine Förderpumpe (64), um Kühl-Schmierstoffemulsion aus einem Tank (60, 60') zum Sensorblock (40) und dem Einmischblock (50) zu führen,
    **dadurch gekennzeichnet, dass** die Vorrichtung des Weiteren eine Bypassleitung (58) umfasst, über die Kühl-Schmierstoffemulsion, die den Sensorblock (40) und den Einmischblock (50) durchlaufen hat, zurück zur Förderpumpe geführt werden kann, wodurch ein von einem Tank (60, 60') abgetrenntes Probenvolumen der Kühl-Schmierstoffemulsion umgewälzt wird, wobei die Vorrichtung (10) zur Durchführung des Verfahrens des Anspruchs 1 eingerichtet ist.

12. Vorrichtung (10) gemäß Anspruch 11, **dadurch gekennzeichnet, dass** diese einen Verbindungsblock (70) umfasst, welcher mit der Förderpumpe (64) in Verbindung steht und eine Vielzahl von Ansaugleitungen (66, 66') für eine Vielzahl von Tanks (60, 60') aufweist, so dass die Vorrichtung selektiv mit jeweils einem Tank (60, 60') aus der Vielzahl von Tanks (60, 60') verbunden werden kann.

13. Vorrichtung (10) gemäß Anspruch 11 oder 12, **dadurch gekennzeichnet, dass** eine Druckluftleitung (68) vorgesehen ist, welche über ein Druckluftventil (82) mit einer Ansaugleitung (66) verbunden ist, so dass in der Ansaugleitung (66) und/oder dem Sensorblock (40) und/oder in dem Einmischblock (50) vorhandene Kühl-Schmierstoffemulsion ausgeblasen werden kann.

14. Vorrichtung (10) gemäß einem der Ansprüche 11 bis 13, **dadurch gekennzeichnet, dass** die Vorrichtung (10) des Weiteren mindestens einen Agitator umfasst, welcher in Strömungsrichtung der Kühl-Schmierstoffemulsion dem Einmischblock (50) nachgeschaltet ist, wobei der Agitator ausgewählt ist aus einem Mischer (26), einem Heizelement, einem Kühlelement, einer Bestrahlungseinheit und Kombinationen aus mehreren dieser Agitatoren.

15. Vorrichtung (10) gemäß einem der Ansprüche 11 bis 14, **dadurch gekennzeichnet, dass** der mindestens eine Sensor (41, 42, 43) ein Refraktometer (200) ist, welcher ein Messfenster (210) aufweist, wobei ein Querschnitt (d1) einer zu dem Messfenster (210) führenden Zulaufleitung (202) derart gewählt ist, dass die Strömungsgeschwindigkeit am Messfenster (210) bei einem vorgegebenen Volumenstrom eine Strömungsgeschwindigkeit im Bereich von 1 bis 500 m/min liegt und die zum Messfenster führende Zulaufleitung (202) zu einer Senkrechten zum Messfenster (210) einen Winkel ($\alpha$) im Bereich von 15 bis 75 ° einschließt.

**Claims**

1.  Method for determining at least one parameter of a cooling lubricant emulsion for machine tools (110, 110'), where a cooling lubricant emulsion is provided in a tank (60, 60') and where the cooling lubricant emulsion is withdrawn from the tank (60, 60'), at least one parameter of the cooling lubricant emulsion is determined using at least one sensor (41, 42, 43) and the cooling lubricant emulsion is returned at least partly to the tank (60, 60'), **characterized in that** the method additionally comprises implementing a measurement wherein a sample is withdrawn from the tank (60, 60'), the sample is passed in a circuit continuously past the at least one sensor (41, 42, 43) to determine at least one parameter of the sample, where continuously or step-wise at least one measurement reagent is added and the at least one parameter is measured continuously until the at least one parameter reaches a predefined threshold, and subsequently at least part of the sample is conveyed back into the tank (60, 60').

2. Method according to Claim 1, **characterized in that** a part of the sample is discarded as waste and not returned to the tank (60, 60'), or **in that** the sample is returned fully to the tank (60, 60').

3. Method according to Claim 1 or 2, **characterized in that**, using an agitator, mixing and/or chemical reaction between the measurement reagent and the cooling lubricant emulsion carried in the circuit is carried out.

4. Method according to any of Claims 1 to 3, **characterized in that** the continuous addition of the measurement reagent is preceded by at least one discontinuous addition of a further measurement reagent.

5. Method according to any of Claims 1 to 4, **characterized in that** the volume of the sample on addition of the measurement reagent and/or of the further measurement reagent is kept constant by withdrawing a part of the sample from the circuit and returning it to the tank (60, 60').

6. Method according to Claim 4 or 5, **characterized in that** a measurement of the total amine/total acid value (TAm/TAc) is carried out, in which case the further measurement reagent is an acid and a base is added continuously as measurement reagent, the at least one parameter being the pH of the cooling lubricant.

7. Method according to any of Claims 1 to 6, **characterized in that** an acid-base titration is carried out for measuring a buffer capacity of the cooling lubricant emulsion, where the measurement reagent is a standard solution selected from an acid or an alkali and the at least one parameter is the pH, or **in that** a metal content of the cooling lubricant emulsion is determined, where the measurement reagent is selected from a complexing agent and the at least one parameter is a light transmittance or a light reflectivity of the cooling lubricant emulsion, or **in that** an oil content of the cooling lubricant emulsion is determined, where the measurement reagent is selected from a concentrated salt solution or an acid and the fraction of the oil phase that forms is recognized by the at least one sensor, or **in that** a nitrite content of the cooling lubricant emulsion is determined and the at least one parameter is a light transmittance of the cooling lubricant emulsion.

8. Method according to any of Claims 1 to 7, **characterized in that** fresh cooling lubricant emulsion is provided by conveying a concentrate from a concentrate reservoir (16) and mixing the concentrate with water, where the concentrate is conveyed from the concentrate reservoir (16) via a concentrate suction withdrawal line (300) with a riser tube (302) and where a filling level (304) of the concentrate in the concentrate reservoir (16) is determined by introducing compressed air into the concentrate suction withdrawal line (300), where an air pressure in the concentrate suction withdrawal line (300) is measured with a pressure sensor (86), a limiting pressure for a pressure increase is determined, and the filling level (304) in the concentrate reservoir (16) is calculated using the limiting pressure.

9. Method according to any of Claims 1 to 8, **characterized in that** the cooling lubricant emulsion is conditioned by adding at least one additive to the cooling lubricant emulsion as a function of the at least one parameter and returning the conditioned cooling lubricant emulsion to the tank (60, 60').

10. Method according to Claim 9, **characterized in that** the additive is undiluted concentrate for producing a cooling lubricant emulsion, the amount of the concentrate being determined as a function of the at least one parameter.

11. Apparatus (10) for determining at least one parameter of a cooling lubricant emulsion for machine tools (110, 110'), comprising a sensor block (40) comprising at least one sensor (41, 42, 43),

> an incorporation block (50) comprising at least one additive pump (51, 52) for incorporating an additive or a measurement reagent by mixing, and
> a conveying pump (64) for passing cooling lubricant emulsion from a tank (60, 60') to the sensor block (40) and to the incorporation block (50),
> **characterized in that** the apparatus further comprises a bypass line (58), via which cooling lubricant emulsion which has passed through the sensor block (40) and the incorporation block (50) can be passed back to the conveying pump, whereby a sample volume of the cooling lubricant emulsion that has been removed from a tank (60, 60') is circulated, the apparatus (10) being configured for implementing the method of Claim 1.

**12.** Apparatus (10) according to Claim 11, **characterized in that** it comprises a connection block (70) which is in communication with the conveying pump (64) and comprises a multiplicity of suction withdrawal lines (66, 66') for a multiplicity of tanks (60, 60'), thus allowing the apparatus to be connected selectively to a respective tank (60, 60') out of the multiplicity of tanks (60, 60').

**13.** Apparatus (10) according to Claim 11 or 12, **characterized in that** a compressed air line (68) is provided which is connected via a compressed air valve (82) to a suction withdrawal line (66), thus allowing cooling lubricant emulsion present in the suction withdrawal line (66) and/or in the sensor block (40) and/or in the incorporation block (50) to be blown out.

**14.** Apparatus (10) according to any of Claims 11 to 13, **characterized in that** the apparatus (10) further comprises at least one agitator which in the flow direction of the cooling lubricant emulsion is downstream of the incorporation block (50), the agitator being selected from a mixer (26), a heating element, a cooling element, an irradiation unit and combinations of two or more of these agitators.

**15.** Apparatus (10) according to any of Claims 11 to 14, **characterized in that** the at least one sensor (41, 42, 43) is a refractometer (200) which has a measuring window (210), where a cross section (d1) of a feed line (202) leading to the measuring window (210) is selected such that the flow rate at the measuring window (210) for a predefined volume flow is a flow rate in the range from 1 to 500 m/min and the feed line (202) leading to the measuring window encloses an angle ($\alpha$) in the range from 15 to 75° with respect to a perpendicular to the measuring window (210).


## Revendications

**1.** Procédé permettant de déterminer au moins un paramètre d'une émulsion de lubrifiant réfrigérant pour machines-outils (110, 110'), dans lequel une émulsion de lubrifiant réfrigérant est fournie dans une cuve (60, 60'), et dans lequel l'émulsion de lubrifiant réfrigérant est prélevé dans la cuve (60, 60'), au moins un paramètre de l'émulsion de lubrifiant réfrigérant est détecté en utilisant au moins un capteur (41, 42, 43), et l'émulsion de lubrifiant réfrigérant est ramenée au moins en partie dans la cuve (60, 60'),

**caractérisé en ce que** le procédé comprend en outre la réalisation d'une mesure dans laquelle un échantillon est prélevé dans la cuve (60, 60'), l'échantillon est en continu amené à passer devant au moins un capteur (41, 42, 43) dans un circuit pour détecter au moins un paramètre de l'échantillon, dans lequel au moins un réactif de mesure est ajouté en continu ou progressivement, et ledit au moins un paramètre est mesuré en continu jusqu'à ce que ledit au moins un paramètre atteigne une valeur seuil prédéfinie, et ensuite au moins une partie de l'échantillon est refoulée vers la cuve (60, 60').

**2.** Procédé selon la revendication 1, **caractérisé en ce qu'**une partie de l'échantillon est rejetée sous forme de déchet et n'est pas ramenée dans la cuve (60, 60'), ou **en ce que** l'échantillon est ramené entièrement dans la cuve (60, 60').

**3.** Procédé selon la revendication 1 ou 2, **caractérisé en ce qu'**à l'aide d'un agitateur, un mélange et/ou une réaction chimique sont effectués entre l'émulsion de lubrifiant réfrigérant acheminée dans le circuit et le réactif de mesure.

**4.** Procédé selon l'une quelconque des revendications 1 à 3, **caractérisé en ce qu'**avant l'ajout continu du réactif de mesure, au moins un ajout discontinu d'un autre réactif de mesure a lieu.

**5.** Procédé selon l'une quelconque des revendications 1 à 4, **caractérisé en ce que** le volume de l'échantillon est maintenu constant lorsque le réactif de mesure et/ou l'autre réactif de mesure sont ajoutés **en ce qu'**une partie de l'échantillon est prélevée dans le circuit et ramenée dans la cuve (60, 60').

**6.** Procédé selon la revendication 4 ou 5, **caractérisé en ce qu'**une mesure de la valeur totale d'amines / valeur totale d'acides (valeur TA/TS) est effectuée, dans lequel l'autre réactif de mesure est un acide et une base est ajoutée en continu comme réactif de mesure, et dans lequel ledit au moins un paramètre est la valeur pH du lubrifiant réfrigérant.

**7.** Procédé selon l'une quelconque des revendications 1 à 6, **caractérisé en ce qu'**une titration acide-base est effectuée pour mesurer une capacité de tampon de l'émulsion de lubrifiant réfrigérant, dans lequel le réactif de mesure est une solution titrante sélectionnée parmi un acide ou une lessive, et ledit au moins un paramètre est la valeur pH, ou **en ce qu'**une teneur en métal de l'émulsion de lubrifiant réfrigérant est déterminée, dans lequel le réactif de mesure est sélectionné parmi un agent complexant, et ledit au moins un paramètre est une transparence ou une réflectance à la lumière de l'émulsion de lubrifiant réfrigérant, ou **en ce qu'**une teneur en huile de l'émulsion de lubrifiant réfrigérant est déterminée, dans lequel le réactif de mesure est sélectionné parmi une solution saline concentrée ou un acide, et ledit au moins un capteur identifie la proportion de la phase huileuse qui se forme, ou **en ce qu'**une teneur en nitrite de l'émulsion de lubrifiant réfrigérant est déterminée, et ledit au moins un paramètre est une transparence à la lumière de l'émulsion de lubrifiant réfrigérant.

8. Procédé selon l'une quelconque des revendications 1 à 7, **caractérisé en ce que** de l'émulsion de lubrifiant réfrigérant fraîche est fournie par le refoulement d'un concentré à partir d'un réservoir de concentré (16) et le mélange du concentré avec de l'eau, dans lequel le concentré est refoulé par une conduite d'aspiration de concentré (300) à l'aide d'un tuyau montant (302) à partir du réservoir de concentré (16), et dans lequel un niveau de remplissage (304) du concentré dans le réservoir de concentré (16) est déterminé par l'introduction d'air comprimé dans la conduite d'aspiration de concentré (300), dans lequel une pression d'air dans la conduite d'aspiration de concentré (300) est mesurée à l'aide d'un capteur de pression (86), une pression limite est déterminée pour une montée en pression, et le niveau de remplissage (304) dans le réservoir de concentré (16) est calculé à l'aide de la pression limite.

9. Procédé selon l'une quelconque des revendications 1 à 8, **caractérisé en ce que** l'émulsion de lubrifiant réfrigérant est conditionnée **en ce qu'**en fonction du au moins un paramètre, au moins un additif est ajouté à l'émulsion de lubrifiant réfrigérant, et l'émulsion de lubrifiant réfrigérant conditionnée est ramenée dans la cuve (60, 60').

10. Procédé selon la revendication 9, **caractérisé en ce que** l'additif est du concentré non dilué pour fabriquer une émulsion de lubrifiant réfrigérant, dans lequel la quantité du concentré est déterminée en fonction du au moins un paramètre.

11. Dispositif (10) permettant de déterminer au moins un paramètre d'une émulsion de lubrifiant réfrigérant pour machines-outils (110, 110') comprenant

   un bloc de capteur (40) comprenant au moins un capteur (41, 42, 43),
   un bloc de mélange (50) comprenant au moins une pompe à additif (51, 52) pour mélanger un additif ou un réactif de mesure, et
   une pompe de refoulement (64) pour acheminer de l'émulsion de lubrifiant réfrigérant d'une cuve (60, 60') au bloc de capteur (40) et au bloc de mélange (50),
   **caractérisé en ce que** le dispositif comprend en outre une conduite de dérivation (58) par laquelle l'émulsion de lubrifiant réfrigérant qui a traversé le bloc de capteur (40) et le bloc de mélange (50) peut être ramenée à la pompe de refoulement, de sorte qu'un volume d'échantillon séparé d'une cuve (60, 60') de l'émulsion de lubrifiant réfrigérant est mis en circulation, dans lequel le dispositif (10) est conçu pour exécuter le procédé selon la revendication 1.

12. Dispositif (10) selon la revendication 11, **caractérisé en ce qu'**il comprend un bloc de raccordement (70) en communication avec la pompe de refoulement (64) et qui présente une pluralité de conduites d'aspiration (66, 66') pour une pluralité de cuves (60, 60') de sorte que le dispositif peut être raccordé sélectivement à respectivement une cuve (60, 60') parmi la pluralité de cuves (60, 60').

13. Dispositif (10) selon la revendication 11 ou 12, **caractérisé en ce qu'**une conduite d'air comprimé (68) est prévue qui est reliée par l'intermédiaire d'une vanne d'air comprimé (82) à une conduite d'aspiration (66) de sorte que de l'émulsion de lubrifiant réfrigérant se trouvant dans la conduite d'aspiration (66) et/ou dans le bloc de capteur (40) et/ou dans le bloc de mélange (50) peut être expulsée.

14. Dispositif (10) selon l'une quelconque des revendications 11 à 13, **caractérisé en ce que** le dispositif (10) comprend en outre au moins un agitateur qui est placé dans la direction d'écoulement de l'émulsion de lubrifiant réfrigérant en aval du bloc de mélange (50), dans lequel l'agitateur est sélectionné parmi un mélangeur (26), un élément chauffant, un élément réfrigérant, une unité d'irradiation et des combinaisons de plusieurs de ces agitateurs.

15. Dispositif (10) selon l'une quelconque des revendications 11 à 14, **caractérisé en ce que** ledit au moins un capteur (41, 42, 43) est un réfractomètre (200) qui présente une fenêtre de mesure (210), dans lequel une section transversale (d1) d'une conduite d'alimentation (202) menant à la fenêtre de mesure (210) est sélectionnée de telle sorte que la vitesse d'écoulement au niveau de la fenêtre de mesure (210) pour un débit volumique prédéfini est une vitesse d'écoulement dans une plage de 1 à 500 m/min et la conduite d'alimentation (202) menant à la fenêtre de mesure forme par rapport à une normale à la fenêtre de mesure (210) un angle ($\alpha$) dans la plage de 15 à 75°.

Fig. 1

EP 3 964 834 B1

Fig. 2

# Fig. 3

Fig. 4

Fig. 5

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- GB 2547056 A
- US 5389546 A
- US 2004159145 A